# EUROPÄISCHE PATENTSCHRIFT

(11) **EP 1 392 169 B1**
(45) Veröffentlichungstag und Bekanntmachung des Hinweises auf die Patenterteilung: **29.03.2006**
(21) Anmeldenummer: 02776504.9
(22) Anmeldetag: 10.05.2002
(51) Int. Cl.: A61B 6/04

(54) **PATIENTENLAGERUNGS- UND TRANSPORTSYSTEM**
PATIENT POSITIONING AND TRANSPORT SYSTEM
SYSTEME DE POSITIONNEMENT ET DE TRANSPORT DE PATIENTS

(30) Priorität: 05.06.2001 DE 10127210
(43) Veröffentlichungstag der Anmeldung: 03.03.2004
(73) Patentinhaber: Deutsches Krebsforschungszentrum, 69120 Heidelberg (DE)
(72) Erfinder: PASTYR, Otto, 69181 Leimen (DE); ECHNER, Gernot, 69257 Wiesenbach (DE); SCHLEGEL, Wolgang, 69118 Heidelberg (DE); STURM, Volker, 69168 Wiesloch-Schatthausen (DE); TREUER, Harald, 50858 Köln (DE)
(74) Vertreter: Weber, Walter
(86) Internationale Anmeldenummer: PCT/EP2002/005129
(87) Internationale Veröffentlichungsnummer: WO 2002/098294

(56) Entgegenhaltungen:
- EP-A- 0 047 958
- US-A- 4 105 923
- US-A- 5 475 884
- US-A- 5 842 987
- US-B1- 6 205 347

## Beschreibung

Die Erfindung betrifft ein Patientenlagerungs- und Transportsystem mit einer transportablen Patientenliege und einem Transportwagen zum Transport der Patientenliege mit Patient, wobei die Patientenliege vom Transportwagen abnehmbar und auf einer Diagnose- oder Behandlungsstation anordenbar ist.

Die der Erfindung zugrundeliegende Problematik besteht darin, daß verschiedene Diagnose- oder Behandlungsstationen jeweils ihre eigenen Patientenliegen aufweisen und Patienten, deren Diagnosen und Behandlungen nacheinander an mehreren Stationen erfolgen müssen, auf Liegen dieser Stationen und wieder zurück auf den Transportwagen gelegt werden müssen. Zwar sind auch Transportwagen mit Patientenliegen bekannt, bei denen die Liege vom Transportwagen abnehmbar ist, um sie auf den Operationstisch zu verbringen, dies löst jedoch noch nicht die Probleme, wenn verschiedenste Diagnose- oder Behandlungsstationen durchlaufen werden müssen, welche verschieden ausgebildet sind, so daß dort die genannte Patientenliege nicht anordenbar ist.

Insbesondere bei stereotaktischen Diagnosen und Behandlungen müssen oft viele Stationen durchlaufen werden. Zunächst muß der Behandlungsbereich in der Regel durch eine bildgebende Einrichtung erfaßt werden, wie die Computertomographie, beispielsweise mit Röntgenaufnahmen, oder die Magnetresonanztomographie (auch Kernspintomographie genannt). Danach erfolgt oftmals eine Angiographie zur Erfassung der Lage von Blutgefäßen. Nach einer anschließenden, manchmal Stunden dauernden stereotaktischen Therapieplanung, bei der die erfaßten Daten im dreidimensionalen Raum ausgewertet und auch die Therapie im dreidimensionalen Raum geplant werden muß, erfolgt die Therapie. Dabei muß der Eingriff räumlich äußerst exakt erfolgen. Solche Behandlungen sind beispielsweise Bestrahlungen, Entnahmen von Biopsien, Einbringung radioaktiver Seeds, Laserstrahlbehandlungen usw.. Diese Diagnosen und Behandlungen erfolgen oftmals als Einzeitbehandlungen, wobei die räumlichen Gegebenheiten der Diagnose durch Bilderfassung bis zum Eingriff mit hoher Präzision reproduzierbar sein müssen, damit das kranke Gewebe durch die Therapie exakt erfaßt und das gesunde Gewebe geschont wird. Befinden sich die Behandlungsbereiche in Nachbarschaft zu Risikoorganen, wie Rückenmark, Nerven usw., so ist oft eine Genauigkeit im Bereich von einem Millimeter erforderlich. Zu diesem Zweck wurden Lagerungs- und Positionssysteme entwickelt, die als Schalen an den Körper angepaßt sind oder als Kopfring am Kopf mittels Haltekloben festgelegt werden. Manchmal erfolgt auch eine Festlegung an Knochen wie den Wirbeln des Rückgrades. Derartige Positionssysteme erhalten auf diese Weise einen festen Bezug zum entsprechenden Körperteil und sind ihrerseits wieder mit Markierungen ausgestattet, die dann erfaßt werden, um eine Lageübereinstimmung bei der Therapie herzustellen.

Bei einer solchen Diagnose und Behandlung mit mehreren Stationen sind Umlagerungen eines Patienten nicht nur zeitraubend und anstrengend für den Patienten und das Personal, sondern sie bergen insbesondere auch die Gefahr in sich, daß ein Positionierungssystem seine Lage zum entsprechenden Körperteil verändert und dadurch der feste Bezug verloren geht. In diesem Fall müßten das Positionierungssystem erneut festgelegt und die gesamte Prozedur beginnend mit der Bildgebung erneut durchlaufen werden. Schlimmer wäre es, wenn ein derartiger Positionierungsfehler nicht bemerkt wird, da dann eine räumliche Ungenauigkeit den Therapieerfolg gefährdet und die Gefahr von Schäden besteht.

Der Erfindung liegt daher die Aufgabe zugrunde, ein Patientenlagerungs- und Transportsystem zu entwickeln, bei dem Patientenumlagerungen zwischen einzelnen Diagnose- oder Behandlungsstationen weitgehendst vermieden werden.

Für ein Patientenlagerungs- und Transportsystem der eingangs genannten Art wird die Aufgabe dadurch gelöst, daß mindestens eine der Patientenliege zuordenbare Adapterplatte vorgesehen ist, wobei die Patientenliege, die Adapterplatte und der Transportwagen miteinander verbindbar und derart ausgebildet sind, daß die Patienten liege wahlweise separat, mit einer oder mit mehreren Adapterplatten abgenommen und an die jeweilige Diagnose- oder Behandlungsstation verbracht werden kann, so daß die Patientenliege mit dem Patienten von dem Transportwagen auf verschiedene, unterschiedlich ausgebildete Diagnose- oder Behandlungsstationen und zurück verbringbar ist.

Der Erfindung liegt die Erkenntnis zugrunde, daß es für die Vielzahl der eingangs genannten Diagnose- oder Behandlungsstationen sowie für ständig neu entwickelte Diagnose- oder Behandlungsstationen nicht ausreicht, wenn nur die Patientenliege von einem Transportwagen abhebbar und beispielsweise auf dem Operationstisch anordenbar ist. Vielmehr muß den verschiedensten Bedingungen an den einzelnen Diagnose- oder Behandlungsstationen Rechnung getragen werden. Dies sind einmal räumliche Bedingungen, der gerätetechnische Aufbau und die jeweilige Konstruktion. Dies sind jedoch oftmals auch physikalische Bedingungen, welche Materialien erfordern, die die Geräte nicht stören. Wegen der vielen unterschiedlichen Anforderungen ist es nicht möglich, eine von einem Transportwagen abnehmbare Patientenliege zu konzipieren, die allen diesen verschiedensten Anforderungen gleichzeitig gerecht wird. Diese Gleichzeitigkeit ist jedoch erforderlich, wenn der Patient mit der Patientenliege ohne Umbetten zumindest alle die Diagnose- oder Behandlungsstationen durchlaufen soll, die zwischen der Bildgebung und der Beendigung der Therapie liegen. Die Erfindung hat deshalb den Weg gewählt, daß der Patientenliege mindestens eine Adapterplatte zugeordnet ist. Das bedeutet, daß die Patientenliege auf einer Adapterplatte ruht und gegebenenfalls darunter noch eine oder mehrere Adapterplatten angeordnet sind. Dies ist alles derart auf dem Transportwagen gelagert, daß die Patientenliege wahlweise separat, mit einer oder mit mehreren Adapterplatten abgenommen und an die jeweilige Diagnose- oder Behandlungsstation verbracht werden kann. Bei der separaten Patientenliege ist dies zweckmäßigerweise die Diagnose- oder Behandlungsstation, in der am wenigsten Raum zur Verfügung steht und/oder die Diagnose- oder Behandlungsstation mit den höchsten Anforderungen an die physikalischen Eigenschaften. Die darunterliegende Adapterplatte kann dagegen einen gewissen Raum in Anspruch nehmen und braucht möglicherweise bezüglich der Materialien, aus denen sie besteht, nicht so strengen Anforderungen gerecht zu werden, wie die Patientenliege. Dafür ist sie dann beispielsweise an gerätetechnische Gegebenheiten weiterer Diagnose- oder Behandlungsstationen angepaßt, wie die Einschubrichtung oder die Mechanismen zur Einbringung der Patientenliege. In dieser Weise können weitere Adapterplatten angeordnet sein, durch die die Patientenliege mit den jeweils dazwischenliegenden Adapterplatten weiteren Diagnose- oder Behandlungsstationen zuordenbar ist, wobei die Ausbildung der jeweiligen Adapterplatte den Gegebenheiten der jeweiligen Diagnose- oder Behandlungsstationen Rechnung tragen. Beispiele hierfür sind bei den jeweiligen Ausgestaltungen und Weiterbildungen der Erfindung erwähnt. Die Erfindung beinhaltet teilweise auch die Anpassung von Diagnose- oder Behandlungsstationen, soweit auf diese Weise eine paßgenaue Anordnung, eine unkomplizierte und erschütterungsfreie Verbringung der Patientenliege auf die Diagnose- oder Behandlungsstation und zurück oder eine zweckmäßige Erreichung der zu erzielenden Kompatibilität möglich ist. Dies kann durch Maßnahmen an der Diagnose- oder Behandlungsstation in Form von Veränderungen oder der Einfügung weiterer Adapterteile erfolgen. Bezüglich solcher Maßnahmen wird ebenfalls auf Weiterbildungen verwiesen.

Ein zweckmäßige Ausgestaltung wurde bereits erwähnt, nämlich, daß mindestens eine der Patientenliege zugeordnete Adapterplatte vorgesehen und die Patientenliege wie die Adapterplatte derart ausgebildet sind, daß die Patientenliege sowohl separat als auch mit Adapterplatte von dem Transportwagen auf verschiedene Diagnose- oder Behandlungsstationen und zurück verbringbar ist. Diese und weitere Möglichkeiten verfolgen den Zweck, eine Kompatibilität zu allen Diagnose- oder Behandlungsstationen einer möglichen Diagnose oder Behandlung herzustellen, da die Erfindung die Einbeziehung der Anforderungen von einer Vielzahl von Diagnose- oder Behandlungsstationen erlaubt. Durch die Adapterplatten wird die geforderte Universalität am zweckmäßigsten erreicht. Selbstverständlich könnte die Patientenliege auch ausschließlich mit Adapterplatten auf Diagnose- oder Behandlungsstationen verbringbar sein.

Zunächst können verschiedene Anforderungen an die Art der Verbringung der Patientenliege in oder auf die jeweilige Diagnose- oder Behandlungsstation bestehen und dementsprechend die Patientenliege und/oder eine der unter dieser angeordneten Adapterplatten ausgebildet sein.

So kann vorgesehen sein, daß mindestens eine der Lagerungen der Patientenliege beziehungsweise der mindestens einen Adapterplatte derart ausgestaltet ist, daß sie eine Verbringung durch Abheben und paßgenaues Aufsetzen ermöglicht. Alternativ kann jedoch auch vorgesehen sein, daß mindestens eine der Lagerungen der Patientenliege beziehungsweise der mindestens einen Adapterplatte derart ausgestaltet ist, daß sie eine Verbringung durch Verschiebung in Längsrichtung ermöglicht oder daß mindestens eine der Lagerungen der Patientenliege beziehungsweise der mindestens einen Adapterplatte derart ausgestaltet ist, sie eine Verbringung durch Verschieben in Querrichtung ermöglicht. Da an den verschiedenen Diagnose- oder Behandlungsstationen unterschiedliche Anforderungen gestellt werden, ist es in Regel zweckmäßig, wenn die Patientenliege mit Adapterplatten derart ausgestaltet ist, daß alle drei der vorgenannten Möglichkeiten der Verbringung der Patientenliege auf eine Diagnose- oder Behandlungsstation in das Patientenlagerungs- und Transportsystem integriert sind.

Zweckmäßigerweise werden die Lagerungen derart ausgestaltet, daß eine feste Verbindung hergestellt werden kann. Beispielsweise kann vorgesehen sein, daß die Patientenliege und mindestens eine Adapterplatte mit Positioniervorrichtungen für definierte unverrückbare Anordnungen ausgestattet sind. Auf diese Weise läßt sich die Patientenliege mit der gewünschten Anzahl von Adapterplatten sowie mit der jeweiligen Diagnose- oder Behandlungsstation fest verbinden, so daß die Positionierung bei Verbringung der Patientenliege mit einer oder mehreren Adapterplatten auf oder in eine Diagnose- oder Behandlungsstation gewährleistet ist. Weiterhin ist es zweckmäßig, daß auch der Transportwagen mit Positioniervorrichtungen für eine definierte unverrückbare Anordnung ausgestattet ist. Dies dient dann dazu, daß der Patient beim Transport in ausreichendem Maß gesichert ist oder daß die Positionierung für den Fall gesichert ist, daß der Transportwagen als solcher in eine Diagnose- oder Behandlungsstation eingefügt wird. Als zusätzliche Sicherung ist es zweckmäßig, wenn die mindestens eine Lagerung, welche ein Abheben der Patientenliege gestattet, eine Sicherung gegen unbeabsichtigtes Abheben aufweist. Besonders beim Transport des Patienten mit dem Transportwagen muß ein solches unbeabsichtigtes Abheben, beispielsweise bei der Überwindung von Niveauunterschieden in Gängen oder an Aufzügen, verhindert werden.

Weitere verschiedene Anforderungen betreffen die Berücksichtigung der physikalischen Gegebenheiten an den verschiedenen Diagnose- oder Behandlungsstationen. Deshalb wird zweckmäßigerweise vorgesehen, daß die Materialien der einzelnen Komponenten des Patientenlagerungs- und Transportsystem derart gewählt sind, daß Störungen von Diagnose- oder Behandlungsstationen ausgeschlossen sind. Dabei müssen immer die Komponenten berücksichtigt werden, die mit den jeweiligen gegenüber solchen Einflüssen empfindlichen Diagnose- oder Behandlungsstationen in Wechselwirkung kommen. Beispielsweise ist es zweckmäßig, wenn der Transportwagen und die mindestens eine Adapterplatte aus nicht magnetischen oder nur sehr schwach magnetischen Materialien besteht. Der Zweck besteht darin, daß bei der Verbringung des Transportwagens mit der Patientenliege und den Adapterplatten an einen Magnetresonanztomographen keine Magnetkräfte wirken, welche Teile in den Magnetresonanztomographen hineinziehen.

Wird vorgesehen, daß die Patientenliege in einem Magnetresonanztomographen angeordnet werden kann, so muß diese aus unmagnetischen und nichtleitenden Materialien bestehen, damit die Bildgebung keine unzulässige Beeinflussung erfährt. In diesem Fall brauchen selbstverständlich die Bestandteile, welche nicht in den Magnetresonanztomographen verbracht werden, diese Eigenschaften nicht aufzuweisen. Die Materialien dieser Bestandteile des Systems müssen nur der Tatsache Rechnung tragen, daß der Transportwagen in die Nähe des Magnetresonanztomographen kommt und dadurch keine Beeinflussung entsteht. Eine weitere Anforderung kann darin bestehen, daß die Patientenliege und die sie tragende Adapterplatte kein Metall enthalten. Diese Ausgestaltung ist beispielsweise dafür gedacht, daß die Patientenliege mit der darunterliegenden Adapterplatte in ein Computertomographiegerät verbracht werden kann und daher diese beiden Komponenten so beschaffen sein müssen, daß sie Röntgenaufnahmen nicht negativ beeinflussen. Für die Anordnung in einem Bestrahlungsgerät darf die Patientenliege oder einer der an diese angeführten Adapterplatten keine Materialien enthalten, die den Behandlungsstrahl beeinflussen. Selbstverständlich sind weitere Anforderungen, beispielsweise an weitere Adapterplatten denkbar, je nach dem, welche Diagnose- oder Behandlungsstationen in Frage kommen und welche Anforderungen dort gestellt werden.

Die vorgenannten Elemente des Patientenlagerungs- und Transportsystems können derart ausgebildet sein, daß sie auf bestehende Diagnose- oder Behandlungsstationen angepaßt sind. Selbstverständlich kann jedoch das Patientenlagerungs- und Transportsystem auch die Ausbildung von Diagnose- oder Behandlungsstationen selbst umfassen, soweit es zweckmäßig ist, diese an die vorgenannten Elemente adaptierbar zu machen, eine leichtere Verbringung der Patientenliege oder eine exaktere Positionierung zu gewährleisten. Deshalb wird vorgeschlagen, daß mindestens eine der Diagnose- oder Behandlungsstationen derart ausgebildet ist, daß eine Adapterplatte unverrückbar anordenbar ist. Dabei kann vorgesehen sein, daß eine Adapterplatte eine Grundplatte ist, welche unverrückbar auf dem Transportwagen anordenbar, jedoch von diesem abhebbar ist. Eine zweckmäßige Ausgestaltung sieht dann vor, daß der Transportwagen derart ausgestaltet ist, daß er mit der Grundplatte über eine Diagnose- oder Behandlungsstation gefahren werden kann, wobei diese eine weitere Lagerung und eine Höhenverstellung aufweist, derart, daß die Grundplatte vom Transportwagen abhebbar und wieder auf diesen aufsetzbar ist, und daß nach einem Abheben der Grundplatte der Transportwagen von dieser Diagnose- oder Behandlungsstation entfembar ist. Zweckmäßigerweise weist dann diese Diagnose- oder Behandlungsstation ebenfalls eine Positioniervorrichtung auf, damit die Positionierung bei der Übergabe nicht verloren geht. Bei der besagten Diagnose- oder Behandlungsstation kann es sich um einen Operationstisch handeln, welcher die genannte Höhenverstellung aufweist. Für die beschriebene Verwendung auf einem Operationstisch kann die Grundplatte wie sonstige Operationstischplatten ausgebildet sein, beispielsweise aus elektrisch leitendem Hartpapier, dies ist ein aus Papierschichten und Harzschichten bestehender Verbundwerkstoff hoher Stabilität, der seine elektrische Leitfähigkeit durch Graphitanteile erhält. Dies ist in bekannter Weise für verschiedene medizinische Behandlungen zweckmäßig. Ist die Zwischenplatte aus Karbon, so gleitet diese sehr gut auf der Grundplatte aus Hartpapier. Die Höhenverstellbarkeit des Operationstisches bietet den zusätzlichen Vorteil der Einstellbarkeit der optimalen Arbeitshöhe für den behandelnden Arzt.

Die Patientenliege kann so ausgebildet sein, daß sie eine längsverschiebbare Lagerung aufweist. Dies ist zweckmäßig, wenn sie Diagnose- oder Behandlungsstationen zugeführt werden soll, die eine entsprechende Lagerung aufweisen, insbesondere, wenn diese als Gegenstück zur längsverschiebbaren Lagerung der Patientenliege ausgebildet ist, derart, daß die Patientenliege vom Transportwagen unmittelbar in die Diagnose- oder Behandlungsstation hineinschiebbar ist. Die längsverschiebbare Lagerung ist zweckmäßigerweise derart ausgebildet, daß ein Abheben der Patientenliege nicht möglich ist. Beispielsweise kann sie an mindestens einer Seite als Schwalbenschwanz- oder T-Nut-Führung ausgebildet sein. Die Diagnose- oder Behandlungsstation, beispielsweise ein Magnetresonanztomograph, weist oft eine röhrenförmige Gestalt auf, in die der Patient eingeschoben werden muß. Dann ist es zweckmäßig, wenn die längsverschiebbare Lagerung mit der Lagerung der Diagnose- oder Behandlungsstation entsprechend abgestimmt ist.

Für die Einbringung in einen Magnetresonanztomographen muß die Patientenliege, wie bereits oben erwähnt, aus unmagnetischen und nichtleitenden Materialien bestehen. Daß die Patientenliege unmittelbar, ohne Adapterplatte in den Magnetresonanztomographen einschiebbar ist, ist deshalb zweckmäßig, weil die Raumverhältnisse dort sehr eng sind und ein hoher Aufbau der einzuschiebenden Patientenliege deshalb unerwünscht ist. Darum ist es auch zweckmäßig, die Patientenliege sehr flach auszugestalten, wobei einer faserverstärkten Sandwichbauweise der Vorzug gegeben wird. Zur Erfüllung dieser räumlichen und der physikalischen Anforderungen kann die Patientenliege aus Keflar® bestehen, vorzugsweise aus zwei härteren Schichten, die einen Kunststoffschaum in der Mitte einschließen. Die Patientenliege derart auszugestalten, daß sie ohne Adapterplatte in den Magnetresonanztomographen einschiebbar ist, hat auch den Grund, daß dann nur die Patientenliege und nicht auch noch eine Adapterplatte den sehr hohen physikalischen Anforderungen gerecht werden muß, nämlich, daß sie absolut unmagnetisch und nichtleitend ist.

Magnetresonanztomographen sind in der Regel bereits mit einer Patientenliege ausgestattet. Damit für die Verwendung der erfindungsgemäßen Patientenliege diese nicht ausgebaut werden muß, wird vorgeschlagen, daß die erfindungsgemäße Patientenliege derart flach gebaut ist, daß sie über die Patientenliege eines Magnetresonanztomographen geschoben werden kann, wobei die Lagerung im Magnetresonanztomographen zu diesem Zweck angeordnet und ausgebildet ist. Sehr oft gibt es an den Patientenliegen der Magnetresonanztomographen bereits an den Rändern längs verlaufende Nuten für Zubehörteile - oft als Schwalbenschwanznuten ausgebildet -, die als Lagerung genutzt werden können. Sind solche Nuten nicht vorhanden, so ist es zweckmäßig bei der Patientenliege des Magnetresonanztomographen, solche Nuten vorzusehen, damit die erfindungsgemäße Patientenliege auf die Patientenliege des Magnetresonanztomographen geschoben werden kann. Selbstverständlich wird auch die genannte separat einschiebbare Patientenliege von der Erfindung umfaßt, da auch diese dadurch gekennzeichnet ist, daß ihr mindestens eine Adapterplatte zugeordnet ist, um sie auch anderen Diagnose- oder Behandlungsstationen zuführen zu können, denn dies ist Voraussetzung, um den erfindungsgemäßen Zweck zu erreichen.

Vorzugsweise wird vorgesehen, daß mindestens eine Positionierungsvorrichtung zur Festlegung von zu behandelnden Körperteilen fest mit der Patientenliege verbindbar ist. Dies ist möglich, da im Unterschied zu den bekannten Patientenliegen eine Umbettung des Patienten nicht mehr erforderlich ist und daher diese Positionierungsvorrichtungen bei der Umbettung nicht mehr auf neue Patientenliegen mitgenommen und dort wieder befestigt werden müssen. Gerade dieses Verbleiben des Patienten mit den Positionierungsvorrichtungen auf der Patientenliege während der gesamten Diagnose und Behandlung, also von der Bildgebung bis zum Ende der Therapie ist der wesentliche Vorzug der erfindungsgemäßen Ausgestaltung einer Patientenliege mit zugeordneten Adapterplatten, die ein solches Umbetten des Patienten überflüssig macht.

Ein Beispiel für eine solche Positionierungsvorrichtung ist ein Kopfhalterungsring, bei dem mittels Haltekloben oder sonstiger Positionierungseinrichtungen der Ring fest am Kopf des Patienten positioniert wird. Um diesen Kopfhalterungsring an der Patientenliege anzuordnen, wird vorgeschlagen, daß am Ende der Patientenliege eine Kopfhalterungsbefestigung angeordnet ist. In diesem Fall kann weiterhin vorgesehen sein, daß die Kopfhalterungsbefestigung als Positionierungsanschlag am Ende einer Längsverschiebung dient, beispielsweise dann, wenn die Patientenliege in einen Magnetresonanztomographen eingeschoben wird. Vorzugsweise wird dann vorgesehen, daß die Kopfhalterungsbefestigung derart angeordnet ist, daß trotz der Lage der Patientenliege über der Patientenliege des Magnetresonanztomographen ein an ihr angeordneter Kopfhalterungsring derart positioniert ist, daß ein in ihm gehaltener Kopf in einem mittleren Bereich des Magnetresonanztomographen zu liegen kommt. Dies hat seinen Grund darin, daß in diesem Bereich die besten und unverzehrten Bilder erzeugt werden können.

Eine Weiterbildung sieht vor, daß zwischen Patientenliege und Grundplatte eine als Zwischenplatte ausgebildete Adapterplatte vorgesehen ist. Diese kann zur Grundplatte eine querverschiebbare Lagerung aufweisen. Dies ist zweckmäßig, wenn ein Patient in Diagnose- oder Behandlungsstationen verbracht werden soll, in denen er quer eingeschoben werden muß. Das ist sehr oft bei Angio- und Computertomographiestationen der Fall, da in vielen Klinikbereichen wenig Platz zur Verfügung steht und darum Geräte mit einer quer verlaufenden Einbringrichtung für Patienten vorgesehen sind. Die querverschiebbare Lagerung besteht zweckmäßigerweise aus Führungen mit einer Sicherung gegen ein Abheben der Zwischenplatte sowie Endanschlägen zur Positionierung. Die Höhe der Führungen ist dann zweckmäßigerweise derart aufeinander abgestimmt, daß die Patientenliege mit Adapterplatte unmittelbar vom Transportwagen in die Diagnose- oder Behandlungsstation einschiebbar ist.

Querverschiebbare Lagerungen sind zweckmäßigerweise derart ausgebildet, daß sie nach einer Seite eine Verschiebung zulassen und nach der anderen Seite Endanschläge aufweisen. Auf diese Weise ist die Patientenliege nach einer Seite gehalten und kann nach der anderen Seite in eine Diagnose- oder Behandlungsstation verbracht werden. Weiterhin ist es zweckmäßig, wenn die querverschiebbare Lagerung V-förmig nach außen weiter und zum Endanschlag sich verjüngende Führungen aufweist. Auf diese Weise kann bei einem Verschieben der Patientenliege ohne weiteres eine Einfädlung von Führungselementen in die Führungen hergestellt werden, ohne daß es erforderlich ist, den Eingang der Führungen aufzusuchen. Eine besonders zweckmäßige Ausgestaltung sieht vor, daß an jeder Seite mindestens zwei Führungen mit Endanschlägen vorgesehen sind, weiterhin, daß die Endanschläge durch ein Zusammenwirken der Endbereiche der Führungen mit Führungs- und Haltebolzen entstehen und zweckmäßigerweise auch, daß durch entfern- und befestigbare Führungs- und Haltebolzen die Zwischenplatte wahlweise nach der einen oder anderen Seite verschiebbar ist. Auf diese Weise läßt sich auswählen, auf welche Seite die Verschiebung stattfinden soll, indem die entfern- und befestigbaren Führungs- und Haltebolzen wahlweise auf der einen oder der anderen Seite befestigt werden.

Die vorgenannten Weiterbildungen sind besonders von Vorteil, wenn eine Diagnose- oder Behandlungsstation derart ausgebildet ist, daß sie mit der Grundplatte der auf dem Transportwagen angeordneten Patientenliege auf einer Höhe liegt und wenn die Diagnose- oder Behandlungsstation ebenfalls mit Führungs- und Haltebolzen derart ausgestattet ist, daß dort die Patientenliege nach der Querverschiebung durch Endanschläge positioniert ist. Auf diese Weise läßt sich eine Querverschiebung auf verschiedene Diagnose- und Behandlungsstationen einrichten, gleichgültig, ob eine Verschiebung nach links oder rechts stattfinden muß. Eine solche Verschiebung ist dann derart, daß von den einen Endanschlägen auf dem Transportwagen eine Verschiebung der Patientenliege zu den anderen Endanschlägen auf der Diagnose- oder Behandlungsstation stattfinden kann.

Weitere Ausgestaltungen der Erfindung betreffen den Transportwagen. So kann vorgesehen sein, daß der Transportwagen an seiner Vorderseite eine portalartige Öffnung aufweist, derart, daß er über eine Diagnose- oder Behandlungsstation gefahren werden kann, damit diese die Patientenliege mit der mindestens einen Adapterplatte übernehmen kann. Bezüglich dieser Übernahme wird auf die oben bereits erwähnte Höhenverstellung einer mit dem Transportwagen zusammenwirkenden Diagnose- oder Behandlungsstation verwiesen.

Der Transportwagen weist zumindest an seinem Kopfende zweckmäßigerweise Federungen auf, welche insbesondere bei Patienten, deren Kopf in einem Kopfhalterungsring festgelegt ist, dazu dienen, ihn vor jeglicher Art von Stößen während des Transports zu schützen. Für eine gute Handhabung des Transportwagens ist es weiterhin zweckmäßig, wenn dieser mit einer Radbremse und mit einer Festlegung in Fahrtrichtung für die vorderen Räder ausgestattet ist, letztere dienen einem sichern zielgerichteten Schieben des Transportwagens. Der Transportwagen kann auch mit einer Höhenverstellung ausgestattet sein, um ihn zur Verbringung der Patientenliege in eine Diagnose- oder Behandlungsstation auf eine Höhe einzustellen, die ein einfaches Verschieben ermöglicht oder auf andere Weise die Verbringung von Liege und Patient erleichtert.

Da es vorkommen kann, daß trotz entsprechender Ausgestaltung der Adapterplatten die Patientenliege trotzdem nicht auf alle Diagnose- oder Behandlungsstationen paßt, kann zusätzlich vorgesehen sein, daß mindestens ein weiteres Adapterteil vorgesehen ist, welches der Anordnung der Patientenliege auf einer Diagnose- oder Behandlungsstation dient, für die keine der Adapterplatten passend ist. Ein derartiges Adapterteil kann beispielsweise der Schaffung einer Auflage auf einer vorhandenen Patientenliege dienen. Der Hintergrund dieser Zusatzeinrichtung besteht darin, daß es Patientenliegen mit völlig verschiedenen Ausgestaltungen, insbesondere unterschiedlich geformten Liegemulden gibt. Da es zu aufwendig wäre, für alle diese Ausgestaltungen von Patientenliegen, insbesondere für derartige Liegemulden Adapterplatten auszubilden, ist es einfacher, wenn für den jeweiligen Zweck ein Adapterteil geschaffen wird, das für diesen Ausgleich sorgt. Dabei kann es sich um ein sehr einfach ausgebildetes Adapterteil handeln, beispielsweise wird vorgeschlagen, daß das Adapterteil aus mindestens zwei Auflagestegen besteht, die durch mindestens eine Stange miteinander verbindbar sind. Damit ist das Adapterteil stabil, einfach aufgebaut und es läßt sich leicht zerlegen und raumsparend verstauen. Es läßt sich auch leicht entsprechend der individuellen Gegebenheiten formen. Die Zwischenfügung eines solchen Adapterteils schließt die Anordnung von Positioniereinrichtungen zwischen der Patientenliege mit Adapterplatten und der Diagnose- oder Behandlungsstation selbstverständlich nicht aus. Vorgeschlagen wird beispielsweise, daß das Adapterteil Führungs- und Haltebolzen aufweist, um die Diagnose- und Behandlungsstation mit Endanschlägen auszustatten. Damit kann eine Verschiebung der Patientenliege vom Transportwagen auf eine Diagnose- oder Behandlungsstation in der oben beschriebenen Art und Weise erfolgen.

Die folgenden Weiterbildungen beziehen sich noch auf weitere Ausgestaltungen des Operationstisches zu dem erfindungsgemäßen Zweck.

Es wird vorgeschlagen, daß der Operationstisch mit einer synchronen elektrischen Höhenverstellung an allen vier Beinen ausgestattet ist und daß eine Einrichtung vorgesehen ist, die nach jeder Betätigung der Höhenverstellung die Stromversorgung vom Netz trennt und diese nur auf ausdrücklichen Befehl wieder herstellt. Dadurch wird verhindert, daß empfindliche elektrische Geräte durch das Vorhandensein einer Spannung gestört werden und es wird auch vermieden, daß die Betätigung der Abtrennung von der Stromversorgung vergessen wird, da diese automatisch erfolgt.

Sowohl für die Aufbringung der Patientenliege als auch für durchzuführende Operationen und sonstige Behandlungsmaßnahmen ist es zweckmäßig, daß der Operationstisch durch eine am Boden angebrachte Standfixierung unverrückbar aber abhebbar gelagert ist. Außerdem wird ein sehr stabiler Aufbau des Operationstisches vorgeschlagen, damit dieser auch eine sichere Positionierung zum Boden gewährleistet.

Selbstverständlich kann der Operationstisch auch noch Einrichtungen enthalten, durch die beispielsweise die Korrektheit der Positionierung des Patienten und/oder der Instrumente überprüft werden kann. Beispielsweise wird vorgeschlagen, daß der Operationstisch mit vertikal- und horizontal angeordneten Röntgenkassettenhaltern ausgestattet ist, so daß es möglich ist, durch entsprechende Röntgenaufnahmen sich nochmals ein Bild von der Positionierung des zu behandelnden Körperteils zu machen.

Soll die Patientenliege mit dem Kopf des Patienten in eine schmal ausgestaltete Diagnose- oder Behandlungsstation eingeführt werden, wie dies manchmal bei einer Gantry oder bei einem C-förmigen Bügel eines Röntgengeräts der Fall ist, so kann es zweckmäßig sein, wenn die Patientenliege sowie die mindestens eine Adapterplatte eine Verjüngung am Kopfende aufweisen.

Die verschiedenen Ausführungsformen der Erfindung können beliebig miteinander kombiniert werden. Dabei können auch eine größere Anzahl oder auch austauschbare Adapterplatten vorgesehen sein. Bei solchen austauschbaren Adapterplatten können die Gegebenheiten der jeweiligen Klinik, also die vorhandenen Geräte berücksichtigt werden und dann die Auswahl der zu liefernden Adapterplatten so getroffen oder individuell angepaßt werden, daß das erfindungsgemäße System mit allen vorhandenen Geräten kompatibel ist. Auch können vorhandene Diagnose- oder Behandlungsstationen an Adapterplatten oder an die Patientenliege angepaßt werden, sofern dies einfacher ist.

Nachfolgend wird die Erfindung anhand eines in der Zeichnung dargestellten Ausführungsbeispiels erläutert. Es zeigen
- **Fig. 1**: ein Ausführungsbeispiel der Erfindung,
- **Fig. 2**: einen Schnitt durch Fig. 1,
- **Fig. 3**: einen Schnitt III-III durch Fig. 2,
- **Fig. 4**: einen Schnitt IV-IV durch Fig. 3,
- **Fig. 5**: die Einbeziehung eines entsprechend ausgebildeten Operationstisches in das erfindungsgemäße System,
- **Fig. 5a**: eine Einzelheit von Fig. 5,
- **Fig. 6**: einen Operationstisch mit Patientenliege und Adapterplatten,
- **Fig. 7**: einen Operationstisch mit einer Zusatzeinrichtung,
- **Fig. 8**: einen Magnetresonanztomographen mit einer erfindungsgemäßen Patientenliege ohne Adapterplatten,
- **Fig. 9**: ein Adapterteil im Schnitt und
- **Fig. 10**: dasselbe in perspektivischer Ansicht

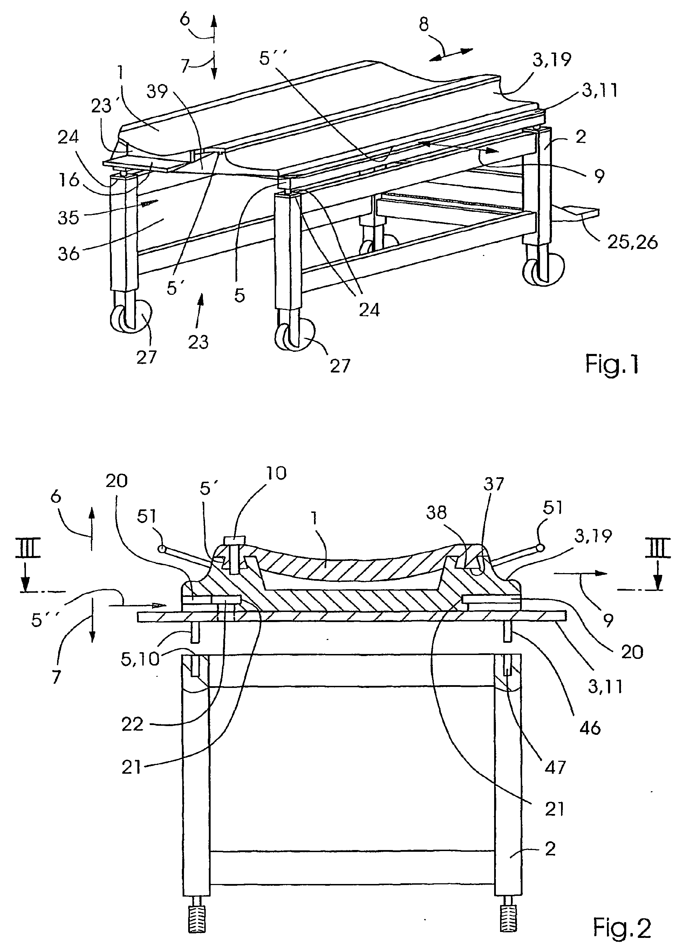

**Fig. 1** zeigt ein Ausfiihrungsbeispiel der Erfindung mit einer auf einem Transportwagen 2 gelagerten Patientenliege 1. Dieser Patientenliege 1 sind zwei Adapterplatten 3 zugeordnet, eine Grundplatte 11 und eine Zwischenplatte 19. Die Patientenliege 1 ist auf der Zwischenplatte 19 derart gelagert, daß sie in Richtung des Pfeils 8 in Längsrichtung verschiebbar ist. Die Zwischenplatte 19 ist wiederum auf der Grundplatte 11 derart gelagert, daß eine Verschiebung in Querrichtung, in Richtung des Pfeils 9, vornehmbar ist. Die Grundplatte 11 ist schließlich auf den Transportwagen 2 in unverrückbarer Weise aufgesetzt, wobei ein Abheben in Richtung des Pfeils 6 und ein Aufsetzen in Richtung des Pfeils 7 möglich ist. Bezüglich der entsprechenden Ausgestaltungen der Lagerungen 5, 5', 5", welche der Verbringung der Patientenliege 1 an verschiedene Diagnose- oder Behandlungsstationen 4 dienen, wird auf die Fig. 2 verwiesen.

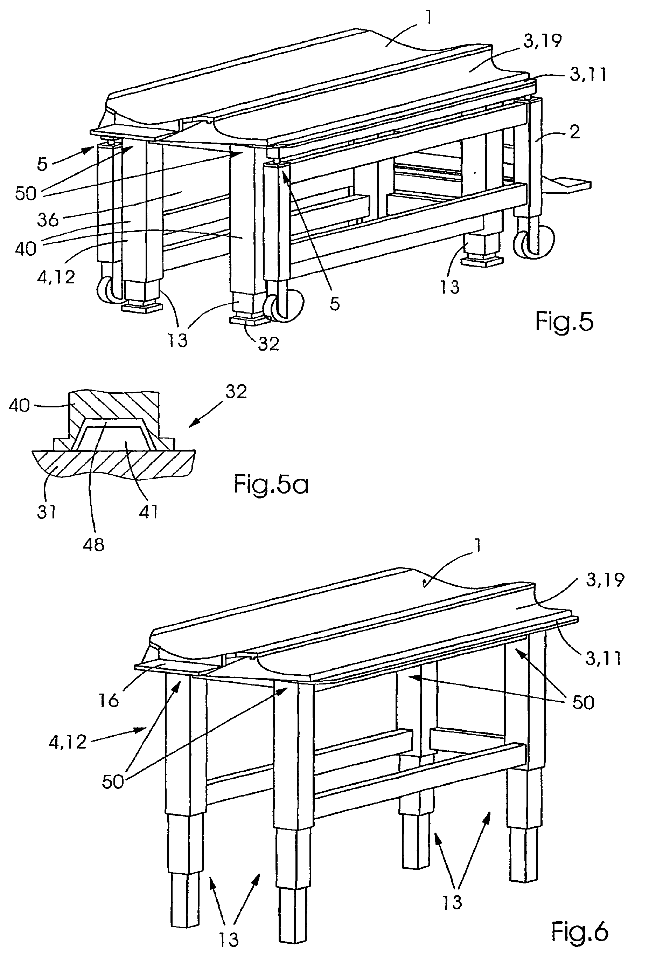

**Fig. 1** zeigt noch Ausgestaltungen des Transportwagens 2, welcher an seiner Vorderseite 35 eine portalartige Öffnung 36 aufweist, die dazu dient, über eine Diagnose- oder Behandlungsstation 4 zu fahren, um dadurch die Patientenliege 1 auf diese Diagnose- oder Behandlungsstation 4 zu verbringen. Diesbezüglich wird auf die Fig. 5 verwiesen. Zumindest am Kopfende 23 des Transportwagens 2 werden zweckmäßigerweise Federungen 24 angeordnet, insbesondere dann, wenn die Patientenliege 1 mit einer Kopfhalterungsbefestigung 16 für einen Kopfhalterungsring 18 ausgestattet ist, wenn also der damit fixierte Patient vor Stößen beim Fahren des Transportwagens 2 geschützt werden muß. Die Kopfhalterungsbefestigung 16 befindet sich am Kopfende 23' der Patientenliege 1, welche vorzugsweise am Kopfende 23 des Transportwagens, also an der Vorderseite 35 angeordnet ist.

**Fig. 2** zeigt einen Schnitt durch die Fig. 1, wobei die Lagerungen 5, 5', 5" sichtbar sind. Die Lagerung der Patientenliege 1 auf der Zwischenplatte 19 ist als längsverschiebbare Lagerung 5' ausgebildet, indem die Patientenliege 1 zwei Gleitschienen 38 aufweist, welche in zwei Nuten 37 der Zwischenplatte 19 laufen. Von den Nuten 37 und Gleitschienen 38 ist vorzugsweise mindestens eine Lagerung 5' als Schwalbenschwanzführung ausgebildet, um ein Abheben 6 der Patientenliege 1 zu vermeiden. Nuten 37 und Gleitschienen 38 sind zweckmäßigerweise an einer Seite als Festlager, also mit minimalstem Spiel und auf der anderen Seite als Loslager mit größerem Spiel ausgebildet, um ein Klemmen zu vermeiden. Damit die Verschiebung in Richtung des Pfeils 8 nicht unbeabsichtigt stattfinden kann, ist eine Positioniervorrichtung 10 vorgesehen, welche beispielsweise als einschiebbarer Stift ausgebildet sein kann, der eine Lage exakt fixiert.

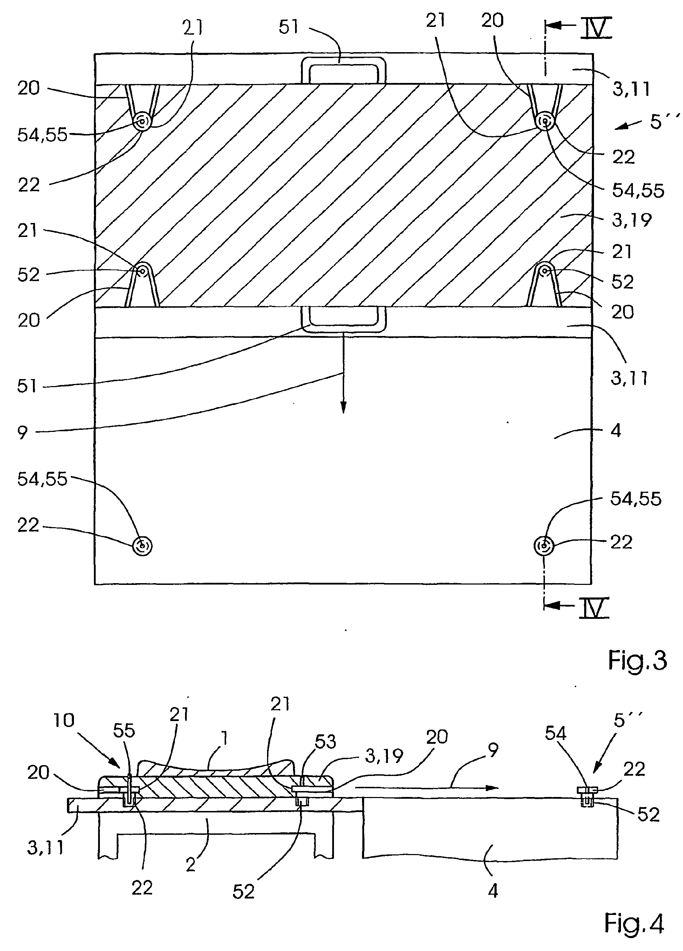

Eine zweite querverschiebbare Lagerung 5" befindet sich zwischen der Zwischenplatte 19 und der Grundplatte 11. Zu diesem Zweck ist die Zwischenplatte 19 mit T-Nuten 20 versehen, in die Führungs- und Haltebolzen 22 einführbar sind, wobei die exakte Positionierung mit der Erreichung von Endanschlägen 21 vollzogen ist. Derartige T-Nuten 20 mit Führungs- und Haltebolzen 22 können zweifach oder auch mehrfach vorgesehen sein. Auch hier ist es zweckmäßig, gegen ein unbeabsichtigtes Verschieben eine Positioniervorrichtung 10 vorzusehen, welche ebenfalls als einsteckbarer Paßstift 55 ausgebildet sein kann. Vorzugsweise wird eine Anordnung gewählt, wie sie in Figur 3 und 4 näher dargestellt ist.

Die beiden Lagerungen 5' und 5" sind zweckmäßigerweise mit entsprechenden Lagerungen 5', 5" von Diagnose- oder Behandlungsstationen 4 abgestimmt, indem diese in der gleichen Höhe und Ausgestaltung ebenfalls Nuten 37 oder T-Nuten 20 aufweisen, so daß sich bei entsprechender Positionierung des Transportwagens 2 der Patient mit der Patientenliege 1 auf die Diagnose- oder Behandlungsstation 4 und zurück verschieben läßt.

Schließlich ist eine abhebbare Lagerung 5 vorgesehen. Diese befindet sich zwischen der Grundplatte 11 und dem Transportwagen 2. Sie kann beispielsweise aus Paßlöchern 47 bestehen, in die Bolzen 46 eingreifen. Letztere können an der Grundplatte 11 angefügt und die Paßlöcher 47 in den Transportwagen 2 eingelassen sein. Selbstverständlich kann dies auch umgekehrt vorgesehen oder eine andere Art der Zusammenfügung gewählt sein. In entsprechender Weise sollte eine weitere, nicht dargestellte Lagerung 50 vorgesehen sein, die in Gegenstücke der Diagnose- oder Behandlungsstationen 4 zur paßgenauen Übergabe eingreifen. Diesbezüglich wird auf Fig. 5 verwiesen.

Die Lagerung 5 ist zweckmäßigerweise mit nicht gezeichneten Sicherungen gegen ein unbeabsichtigtes Abheben 6 ausgestattet, um ein solches beim Transport in Folge von Bodenunebenheiten zu vermeiden.

Die **Fig. 3** zeigt einen Schnitt III-III durch die Fig. 2, um eine zweckmäßige Ausgestaltung einer längsverschiebbaren Halterung 5' darzustellen. Dabei sind die Führungen 20 V-förmig ausgebildet, wobei ein breiter Einlaß geformt ist und sich die Führungen 20 bis zu den Endanschlägen 21 verjüngen. Diese Führungen 20 sind als T-Nuten ausgebildet und wirken mit Führungs- und Haltebolzen 22 zusammen, welche eine komplementäre pilzartige Form aufweisen. Die Endanschläge 21 werden dadurch gebildet, daß die Führungs- und Haltebolzen 22 an den Enden der Führungen 20 anschlagen.

In diesem Ausführungsbeispiel sind vier Führungen 20 vorgeschlagen, an jeder Seite zwei. Selbstverständlich können jedoch auch mehrere Führungen 20 vorgesehen sein.

Die Funktionsweise ist hier dadurch ersichtlich, daß der Schnitt genau an der Oberseite der T-förmigen Nut 20 vorgenommen wurde. Dadurch ist zu sehen, wie die Führungs- und Haltebolzen 22 mit den Enden der T-Nuten 20 die Endanschläge 21 bilden. Dabei sind die Führungs- und Haltebolzen 22 an der entgegengesetzten Seite zu der Verschiebungsrichtung 9 angeordnet, die erforderlich ist, um die Patientenliege 1 auf eine Diagnose- oder Behandlungsstation 4 zu verbringen. Diese Diagnose- oder Behandlungsstation 4 weist zweckmäßigerweise ebenfalls Führungs- und Haltebolzen 22 auf, welche wiederum als Endanschläge 21 dienen, wenn die Patientenliege 1 durch ein Verschieben 9 auf der Grundplatte 11 zur Diagnose- oder Behandlungsstation 4 dort in ihrer Endposition angelangt ist. Auf diese Weise wird die Patientenliege 1 von einer definierten Position auf dem Transportwagen 2 in die definierte Position auf der Diagnose- oder Behandlungsstation 4 verbracht.

Zum Auswechseln der Führungs- und Haltebolzen 22 sind diese in Gewinde 52 eingeschraubt, welche beidseitig in die Grundplatte 11 eingefügt sind. Auf diese Weise kann je nach Ausgestaltung und Aufstellung der Diagnose- oder Behandlungsstation 4 die Patientenliege 1 nach links oder nach rechts vom Transportwagen 2 geschoben werden. Weiterhin sind noch Paßlöcher 54 für Paßstifte 55 sichtbar, welche in die Führungs- und Haltebolzen 22 eingearbeitet sind. Griffe 51 an jeder Seite der Zwischenplatte 19 dienen dem Zweck, daß diese dort ergriffen werden kann, um sie in der gezeichneten oder in der umgekehrte Richtung zur Diagnose- oder Behandlungsstation 4 zu verschieben.

**Fig. 4** zeigt dieselbe Anordnung als Schnitt IV-IV. Dabei ist der Bereich zwischen der Zwischenplatte 19 und der Patientenliege 1 vereinfacht dargestellt. Hier ist ersichtlich, wie die Führungs- und Haltebolzen 22 die Endanschläge 21 auf dem Transportwagen 2 bilden. Mindestens ein Paßstift 55 ist als Sicherung für den Transport eingebracht, wobei er durch Paßlöcher 53 der Zwischenplatte 19 hindurch in die Paßlöcher 54 der Führungs- und Haltebolzen 22 gesteckt ist. Wird dieser Paßstift 55, der als Positioniervorrichtung 10 dient, entfernt, so kann die Zwischenplatte 19 mit der Patientenliege 1 auf die Diagnose- oder Behandlungsstation 4 verschoben werden, bis die Führungs- und Haltebolzen 22 der Diagnose- oder Behandlungsstation 4 auf der rechten Seite der Zwischenplatte 19 in die Führungen 20 eingreifen und dort am Ende dieser Führungen 20 die Endanschläge 21 bilden. Auch an der Diagnose- oder Behandlungsstation 4. lassen sich Paßstifte 55 zur Positionierung 10 einstecken. Selbstverständlich muß die Höhe der Grundplatte 11 exakt auf die Höhe der Diagnose- oder Behandlungsstation 4 oder umgekehrt eingestellt sein.

**Fig. 5** zeigt die Einbeziehung eines entsprechend ausgebildeten Operationstisches 12 in das erfindungsgemäße System. Der zuvor beschriebene Transportwagen 2 mit der Patientenliege 1 und den Adapterplatten 3 wird mit Hilfe der portalartigen Öffnung 36 über den Operationstisch 12 gefahren. Danach wird die in den Beinen 40 des Operationstisches 12 integrierte Höhenverstellung 13 betätigt und dadurch die Grundplatte 11 mit der Zwischenplatte 19 und der Patientenliege 1 von dem Transportwagen 2 abgehoben, wie dies bereits in Fig. 2 dargestellt ist. Für diesen Zweck ist vorzugsweise eine weitere Lagerung 50 vorgesehen, die in den Operationstisch 12 und die Grundplatte 11 integriert ist, damit letztere bei der Übernahme sicher fixiert ist. Wenn die Höhenverstellung derart erfolgt ist, daß die Bolzen 46 aus den Paßlöchern 47 des Transportwagens 2 herausbewegt sind und die Lagerung 50 in entgegengesetzter Weise eingegriffen hat, kann der Transportwagen 2 entfernt werden und es ist möglich, die Patientenliege 1 auf dem Operationstisch 12 in beliebiger Höhe einzustellen. Der Operationstisch 12 ist zweckmäßigerweise auf dem Boden 31 mittels einer Standfixierung 32 fixiert, welche als Einzelheit in **Fig. 5a** dargestellt ist. Dort greift eine am Boden befestigte Fixierung 41 in eine Aushöhlung 48 der Beine 40 des Operationstisches 12 ein.

**Fig. 6** zeigt den Operationstisch 12 mit einer Patientenliege 1, die mittels der Grundplatte 11 und der weiteren Lagerung 50 auf diesem exakt fixiert ist. Dabei dient die Höhenverstellung 13 der Einstellung der Patientenliege 1 in die für den behandelnden Arzt optimale Höhenlage. Dies ist ein zusätzlicher Nutzen, der bereits oben beschriebenen Höhenverstellung 13. Die exakte Positionierung des Patienten auf dem Operationstisch 12 ist besonders bei Eingriffen wichtig, bei denen mittels eines stereotaktischen Handhabungsgeräts, z. B. eines eingestellten Armes, ein definierter Zielpunkt aus einer definierten Einbringungsrichtung angefahren werden muß.

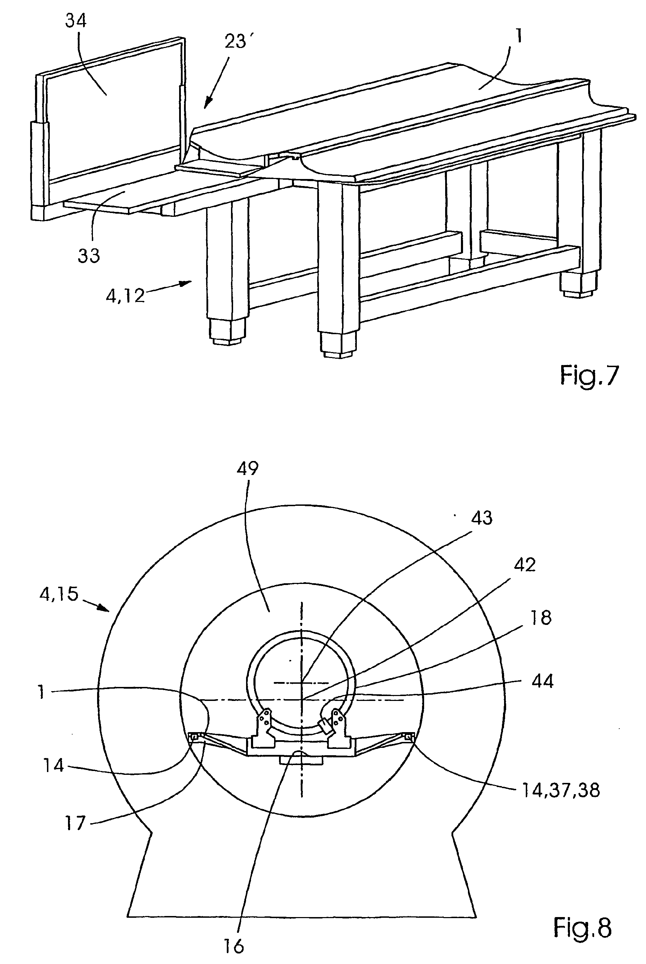

**Fig. 7** zeigt einen Operationstisch 12 mit einer Zusatzeinrichtung. Hier sind am Kopfende 23' ein vertikaler Röntgenkassettenhalter 33 und ein horizontaler Röntgenkassettenhalter 34 angebracht, um mittels einer Röntgenaufnahme nochmals die exakte Lage des Patienten und/oder der Instrumente vor der Vornahme eines Eingriffs zu verifizieren. Diese Röntgenkassettenhalter 33, 34 sind für Aufnahmen im Kopfbereich ausgebildet. Selbstverständlich können sie auch für andere Körperbereiche eingesetzt werden. Entsprechend ist dann ihre Größe und Anbringung.

**Fig. 8** zeigt einen Magnetresonanztomographen 15 mit einer röhrenförmigen Öffnung 49, in welchen eine Patientenliege 1 eingebracht ist. Diese Patientenliege 1 verfügt über Gleitschienen 38, die in entsprechenden Nuten 37 laufen. Dabei entspricht die längsverschiebbare Lagerung 5' des Transportwagen 2 zweckmäßigerweise einer entsprechenden Lagerung 14 des Magnetresonanztomographen 15, derart, daß die Patientenliege 1 unmittelbar vom Transportwagen 2 in den Magnetresonanztomographen 15 eingeschoben werden kann. Auch hier ist mindestens eine Schwalbenschwanzführung zweckmäßig. Aus oben genannten Gründen wird hier die Patientenliege 1 separat eingeschoben, zumal im dargestellten Fall sich in dem Magnetresonanztomographen 15 eine schon dort angeordnete Patientenliege 17 befindet, welche für das Einbringen der Patientenliege 1 nicht ausgebaut werden soll. Durch die geringe Höhe der Patientenliege 1, welche ohne Adapterplatte 3 eingefügt ist, findet nur eine geringe Erhöhung der Lage des Patienten statt, so daß es möglich ist, eine Kopfhalterungsbefestigung 16 mit einem Kopfhalterungsring 18 derart anzuordnen, daß die Mitte 43 des Kopfhalterungsrings 18 im Bereich des Zentrums 42 des Magnetresonanztomographen 15 liegt. Dargestellt ist dabei, daß hier lediglich ein geringer Abstand zwischen den beiden Zentren 42 und 43 auftritt, so daß es noch zu einer exakten Abbildung kommt und der Kopf nicht außerhalb dieses Bereichs der exakten Abbildung liegt. Dargestellt sind noch Haltekloben 44 zur Fixierung eines Kopfes, welche in bekannter Weise ausgebildet sind.

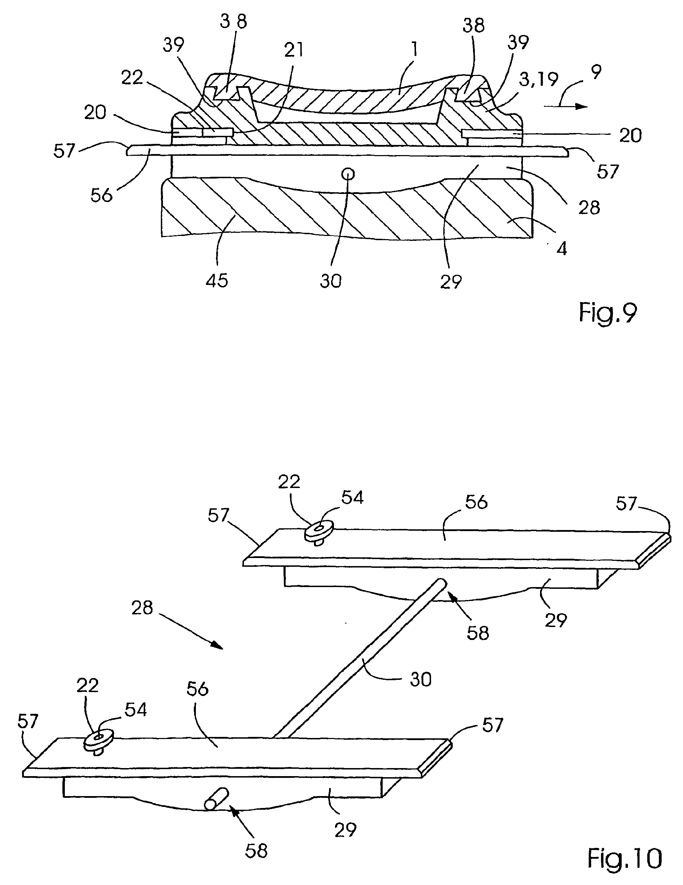

**Fig. 9** zeigt ein Adapterteil 28 im Schnitt. Dies ist insbesondere zweckmäßig, wenn Diagnose- oder Behandlungsstationen 4 vorhandene Patientenliegen 45 aufweisen, die bezüglich ihrer Formgebung sehr unterschiedlich ausgestaltet sein können. In diesem Fall ist das Adapterteil 28 aus mindestens zwei Auflagestege 29 ausgebildet, welche mit mindestens einer Stange 30 verbunden sind, wie dies in perspektivischer Ansicht in **Fig. 10** dargestellt ist. Auf dieses Adapterteil 29 kann die Zwischenplatte 19 unmittelbar oder vorzugsweise - wie dargestellt - auf ein Lagerbrett 56 aufgelegt werden, wobei auch hier Positioniervorrichtungen 10 zur exakten Positionierung vorgesehen sein können. Sowohl die Positioniereinrichtung 10 wie die Lagerung 5" entspricht der Beschreibung zu Fig. 3 und 4 mit dem Unterschied, daß hier die Diagnose- und Behandlungsstation 4 nicht unmittelbar, sondern über das Adapterteil 28 mit den Führungs- und Haltebolzen 22 ausgestattet ist. Die übrigen Funktionen sind mit den zu Fig. 3 und 4 beschriebenen identisch. Weiterhin sind die Auflagestege 29 mit Klemmvorrichtungen 58 ausgestattet, um sie auf der mindestens einen Stange 30 beliebig positionieren und dann festlegen zu können. Weiterhin dienen Fasern 57 der Lagebretter 56 einem guten Gleiten der Zwischenplatte 19 ohne Hemmung durch eine überstehende Kante. Selbstverständlich können auch mehrere Auflagestege 29 angeordnet sein. Der Vorteil dieses Adapterteils 28 besteht darin, daß die Auflagestege 29 ohne Schwierigkeiten entsprechend geformt werden können, wobei sie der unterschiedlichen Formgebung von vorhandenen Patientenliegen 45 anpaßbar sind.

Die Darstellungen zeigen lediglich eine kleine, bevorzugte Auswahl von Ausgestaltungsmöglichkeiten der Erfindung. Wesentlich ist dabei, daß die Ausbildung der Patientenliege 1 und der Adapterplatten 3 von den verschiedenen Diagnose- oder Behandlungsstationen 4 abhängt:

Wie bereits dargestellt, wird auf einen Operationstisch 12 die Patientenliege 1 mit Zwischenplatte 19 und Grundplatte 11 aufgebracht, wobei eine abhebbare Lagerung 5, 50 am Transportwagen 2 und am Operationstisch 12 vorgesehen sind.

Beim Magnetresonanztomographen 15 wird nur die Patientenliege 1 mittels einer längsverschiebbaren Lagerung 5" eingeschoben, wobei das Material der Patientenliege 1 absolut unmagnetisch und nichtleitend sein muß.

Bei einem Computertomographiegerät und einem Angiographiegerät erfolgt in der Regel eine Quereinschiebung der Patientenliege 1 mit der Zwischenplatte 19. Vorraussetzung für die Einbringung in ein Computertomographiegerät ist, daß die vorgenannten Bestandteile 1, 19 kein Metall aufweisen. Diese Geräte verfügen dann zweckmäßigerweise über entsprechende querverschiebbare Lagerungen 5", um die Zwischenplatte 19 mit der Patientenliege 1 unmittelbar in die Geräte einschieben zu können. Zu diesem Zweck und für die Einfügung in weitere Diagnose- oder Behandlungsstationen 4, wie dem Magnetresonanztomographen 15, kann der Transportwagen 2 auch eine Höhenverstellung aufweisen.

In ein Bestrahlungsgerät wird die Patientenliege 1 in der Regel wie beim Operationstisch 12 mit Grundplatte 11 und Zwischenplatte 19 eingefügt, wobei auch hier jeweils abhebbare Lagerungen 5 vorgesehen sind.

Dies ist selbstverständlich nur eine beispielhafte Aufzählung von Diagnose- oder Behandlungsstationen 4 und Lagerungen 5, 5', 5", 50 weitere Diagnose- oder Behandlungsstationen 4 und auch andere Ausgestaltungen der vorgenannten Lagerungen 5, 5', 5", 50 sind denkbar, um sie an vorhandene Diagnose- oder Behandlungsstationen 4 anzupassen und es ist weiterhin möglich, weitere Diagnose- oder Behandlungsstationen 4 ebenfalls derart anzupassen, daß Verschiebungen der Patientenliege 1 oder einer der Adapterplatten 3 von dem Transportwagen 2 unmittelbar in die jeweilige Diagnose- oder Behandlungsstation 4 möglich sind und dabei Anschläge und weitere Positioniervorrichtungen für die jeweilige exakte Positionierung sorgen.

Selbstverständlich können und sollten noch Markierungen an Fixiervorrichtungen, wie beispielsweise dem Kopfhalterungsring 18 oder an angepaßten Schalen für die entsprechenden Körperteile vorgesehen sein, um in den einzelnen Diagnose- oder Behandlungsstationen 4 die Positionierung überprüfen und eine Feinpositionierung vornehmen zu können. Diesbezüglich wird auf entsprechende bekannte Methoden und Einrichtungen verwiesen.

### Bezugszeichenliste

- 1: Patientenliege
- 2: Transportwagen
- 3: Adapterplatten
- 4: Diagnose- oder Behandlungsstationen
- 5, 5', 5": Lagerungen
- 5: abhebbare Lagerung
- 5': längsverschiebbare Lagerung
- 5": querverschiebbare Lagerung
- 6: Pfeil: Abheben
- 7: Pfeil: Aufsetzen
- 8: Pfeil: Verschieben in Längsrichtung
- 9: Pfeil: Verschieben in Querrichtung
- 10: Positioniervorrichtungen
- 11: Grundplatte
- 12: Operationstisch
- 13: Höhenverstellung
- 14: Lagerung ein einem Magnetresonanztomographen
- 15: Magnetresonanztomograph
- 16: Kopfhalterungsbefestigung
- 17: Patientenliege eines Magnetresonanztomographen
- 18: Kopfhalterungsring
- 19: Zwischenplatte
- 20: Führungen (T-Nut)
- 21: Endanschläge
- 22: Führungs- und Haltebolzen
- 23: Kopfende des Transportwagens
- 23': Kopfende der Patientenliege
- 24: Federungen
- 25: Radbremse
- 26: Festlegung für die vorderen Räder
- 27: vordere Räder
- 28: Adapterteil
- 29: Auflagestege
- 30: Stange
- 31: Boden
- 32: Standfixierung
- 33: vertikale Röntgenkassettenhalter
- 34: horizontaler Röntgenkassettenhalter
- 35: Vorderseite des Transportwagens
- 36: portalartige Öffnung
- 37: Nut
- 38: Gleitschiene
- 39: Verjüngung
- 40: Bein des Operationstisches
- 41: am Boden befestigte Fixierung
- 42: Zentrum des Magnetresonanztomographen
- 43: Mitte des Kopfhalterungsrings
- 44: Haltekloben zur Fixierung eines Kopfes
- 45: vorhandene Patientenliege einer Diagnose- oder Behandlungsstation
- 46: Bolzen
- 47: Paßlöcher
- 48: Aushöhlung
- 49: röhrenförmige Öffnung
- 50: weitere Lagerung
- 51: Griffe
- 52: Gewinde für Führungs- und Haltebolzen
- 53: Paßlöcher für Paßstifte
- 54: Paßlöcher in Führungs- und Haltebolzen
- 55: Paßstifte
- 56: Lagerbrett
- 57: Fase
- 58: Klemmvorrichtung

## Patentansprüche

1. Patientenlagerungs- und Transportsystem mit einer transportablen Patientenliege (1) und einem Transportwagen (2) zum Transport der Patientenliege (1) mit Patient, wobei die Patientenliege (1) vom Transportwagen (2) abnehmbar und auf einer Diagnose- oder Behandlungsstation (4) anordenbar ist,
**dadurch gekennzeichnet,**
**daß** mindestens eine der Patientenliege (1) zuordenbare Adapterplatte (3) vorgesehen ist, wobei die Patientenliege (1), die Adapterplatte (3) und der Transportwagen (2) miteinander verbindbar und derart ausgebildet sind, daß die Patientenliege wahlweise separat, mit einer oder mit mehreren Adapterplatten abgenommen und an die jeweilige Diagnose- oder Behandlungsstation verbracht werden kann, so daß die Patientenliege (1) mit dem Patienten von dem Transportwagen (2) auf verschiedene, unterschiedlich ausgebildete Diagnose- oder Behandlungsstationen (4) und zurück verbringbar ist.

2. System nach Anspruch 1,
**dadurch gekennzeichnet,**
**daß** die Patientenliege (1) derart ausgestaltet ist, daß sie separat auf mindestens eine Diagnose- oder Behandlungsstation (4) und zurück verbringbar ist.

3. System nach Anspruch 2,
**dadurch gekennzeichnet,**
**daß** die mindestens eine Adapterplatte (3) derart ausgestaltet ist, daß mit ihr die Patientenliege (1) auf mindestens eine weitere Diagnose- oder Behandlungsstation (4) und zurück verbringbar ist.

4. System nach einem der Ansprüche 1 bis 3,
**dadurch gekennzeichnet,**
**daß** mindestens eine der Lagerungen (5) der Patientenliege (1) beziehungsweise der mindestens einen Adapterplatte (3) derart ausgestaltet ist, daß sie eine Verbringung durch Abheben (6) und paßgenaues Aufsetzen (7) ermöglicht.

5. System nach einem der Ansprüche 1 bis 4,
**dadurch gekennzeichnet,**
**daß** mindestens eine der Lagerungen (5') der Patientenliege (1) beziehungsweise der mindestens einen Adapterplatte (3) derart ausgestaltet ist, daß sie eine Verbringung durch Verschieben in Längsrichtung (8) ermöglicht.

6. System nach einem der Ansprüche 1 bis 5,
**dadurch gekennzeichnet,**
**daß** mindestens eine der Lagerungen (5") der Patientenliege (1) beziehungsweise der mindestens einen Adapterplatte (3) derart ausgestaltet ist, daß sie eine Verbringung durch Verschieben in Querrichtung (9) ermöglicht.

7. System nach einem der Ansprüche 1 bis 6,
**dadurch gekennzeichnet,**
**daß** die Patientenliege (1) und die mindestens eine Adapterplatte (3) mit Positioniervorrichtungen (10) für definierte unverrückbare Anordnungen ausgestattet sind.

8. System nach Anspruch 7,
**dadurch gekennzeichnet,**
**daß** auch der Transportwagen (2) mit Positioniervorrichtungen (10) für eine definierte unverrückbare Anordnung ausgestattet ist.

9. System nach einem der Ansprüche 4 bis 8,
**dadurch gekennzeichnet,**
**daß** die mindestens eine Lagerung (5, 5', 5") eine Sicherung gegen unbeabsichtigtes Abheben (6) aufweist.

10. System nach einem der Ansprüche 1 bis 9,
**dadurch gekennzeichnet,**
**daß** die Materialien seiner einzelnen Komponenten (1, 2, 3) derart gewählt sind, daß Störungen der Diagnose- oder Behandlungsstationen (4), mit denen diese Komponenten (1, 2, 3) in Wechselwirkung kommen, ausgeschlossen sind.

11. System nach Anspruch 10,
**dadurch gekennzeichnet,**
**daß** der Transportwagen (2) und die mindestens eine Adapterplatte (3) aus unmagnetischen oder nur sehr schwach magnetischen Materialien bestehen.

12. System nach Anspruch 10 oder 11,
**dadurch gekennzeichnet,**
**daß** die Patientenliege (1) aus unmagnetischen und nichtleitenden Materialien besteht.

13. System nach Anspruch 10,11 oder 12,
**dadurch gekennzeichnet,**
**daß** die Patientenliege (1) und die sie tragende Adapterplatte (3) kein Metall enthalten.

14. System nach einem der Ansprüche 1 bis 13,
**dadurch gekennzeichnet,**
**daß** mindestens eine der Diagnose- oder Behandlungsstationen (4) derart ausgebildet ist, daß eine Adapterplatte (3) unverrückbar anordenbar ist.

15. System nach einem der Ansprüche 1 bis 14,
**dadurch gekennzeichnet,**
**daß** eine Adapterplatte (3) eine Grundplatte (11) ist, welche unverrückbar auf dem Transportwagen (2) anordenbar, jedoch von diesem abhebbar ist.

16. System nach Anspruch 14 und 15,
**dadurch gekennzeichnet,**
**daß** der Transportwagen (2) derart ausgestaltet ist, daß er mit der Grundplatte (11) über eine Diagnose- oder Behandlungsstation (4) gefahren werden kann, wobei diese eine weitere Lagerung (50) und eine Höhenverstellung (13) aufweist, derart, daß die Grundplatte (11) mit der Patientenliege (1) vom Transportwagen (2) abhebbar (6) und wieder auf diesen aufsetzbar (7) ist, und daß nach einem Abheben (6) der Grundplatte (11) der Transportwagen (2) von dieser Diagnose- oder Behandlungsstation (4) entfembar ist.

17. System nach Anspruch 14, 15 oder 16,
**dadurch gekennzeichnet,**
**daß** diese Diagnose- oder Behandlungsstation (4) ein Operationstisch (12) ist.

18. System nach einem der Ansprüche 1 bis 17,
**dadurch gekennzeichnet,**
**daß** die Patientenliege (1) eine längsverschiebbare Lagerung (5') aufweist.

19. System nach Anspruch 18,
**dadurch gekennzeichnet,**
**daß** die längsverschiebbare Lagerung (5') derart ausgebildet ist, daß ein Abheben (6) der Patientenliege (1) nicht möglich ist.

20. System nach Anspruch 18 oder 19,
**dadurch gekennzeichnet,**
**daß** eine Diagnose- oder Behandlungsstation (4) eine Lagerung aufweist, welche als Gegenstück zur längsverschiebbaren Lagerung (5') der Patientenliege (1) ausgebildet ist, derart, daß die Patientenliege (1) vom Transportwagen (2) unmittelbar in die Diagnose- oder Behandlungsstation (4) hineinschiebbar ist.

21. System nach Anspruch 20,
**dadurch gekennzeichnet,**
**daß** die längsverschiebbare Lagerung (5') mit einer Lagerung (14) in einem Magnetresonanztomographen (15) entsprechend abgestimmt ist.

22. System nach Anspruch 21,
**dadurch gekennzeichnet,**
**daß** die Patientenliege (1) derart flach gebaut ist, daß sie über die Patientenliege (17) eines Magnetresonanztomographen (15) geschoben werden kann, wobei die Lagerung (14) im Magnetresonanztomographen (15) zu diesem Zweck angeordnet und ausgebildet ist.

23. System nach einem der Ansprüche 1 bis 22,
**dadurch gekennzeichnet,**
**daß** mindestens eine Positioniervorrichtung zur Festlegung von zu behandelnden Körperteilen fest mit der Patientenliege (1) verbindbar ist.

24. System nach Anspruch 23,
**dadurch gekennzeichnet,**
**daß** am Ende der Patientenliege (1) eine Kopfhalterungsbefestigung (16) angeordnet ist.

25. System nach Anspruch 24,
**dadurch gekennzeichnet,**
**daß** die Kopfhalterungsbefestigung (16) gleichzeitig als Positionierungsanschlag am Ende einer Längsverschiebung (8) dient.

26. System nach Anspruch 24 oder 25,
**dadurch gekennzeichnet,**
**daß** die Kopfhalterungsbefestigung (16) derart angeordnet ist, daß trotz der Lage der Patientenliege (1) über der Patientenliege (17) des Magnetresonanztomographen (15) ein an ihr angeordneter Kopfhalterungsring (18) derart positioniert ist, daß ein in ihm gehaltener Kopf in einem mittleren Bereich des Magnetresonanztomographen (15) zu liegen kommt.

27. System nach einem der Ansprüche 1 bis 26,
**dadurch gekennzeichnet,**
**daß** zwischen Patientenliege (1) und Grundplatte (11) eine als Zwischenplatte (19) ausgebildete Adapterplatte (3) vorgesehen ist.

28. System nach Anspruch 27,
**dadurch gekennzeichnet,**
**daß** die Zwischenplatte (19) zur Grundplatte (11) eine querverschiebbare Lagerung (5") aufweist.

29. System nach Anspruch 28,
**dadurch gekennzeichnet,**
**daß** die querverschiebbare Lagerung (5") aus Führungen (20) mit einer Sicherung gegen ein Abheben (6) der Zwischenplatte (19) sowie Endanschlägen (21) zur Positionierung besteht.

30. System nach Anspruch 28 oder 29,
**dadurch gekennzeichnet,**
**daß** die querverschiebbare Lagerung (5") nach einer Seite eine Verschiebung (9) zuläßt und nach der anderen Seite Endanschläge (21) aufweist.

31. System nach Anspruch 28, 29 oder 30,
**dadurch gekennzeichnet,**
**daß** die querverschiebbare Lagerung (5") V-förmig nach außen weite und zum Endanschlag (21) sich verjüngende Führungen (20) aufweist.

32. System nach einem der Ansprüche 28 bis 31,
**dadurch gekennzeichnet,**
**daß** an jeder Seite mindestens zwei Führungen (20) mit Endanschlägen (21) vorgesehen sind.

33. System nach Anspruch 32,
**dadurch gekennzeichnet,**
**daß** die Endanschläge (21) durch ein Zusammenwirken der Endbereiche der Führungen (20) mit Führungs- und Haltebolzen (22) entstehen.

34. System nach Anspruch 33,
**dadurch gekennzeichnet,**
**daß** durch entfern- und befestigbare Führungs- und Haltebolzen (22) die Zwischenplatte (19) wahlweise nach der einen oder anderen Seite verschiebbar (9) ist.

35. System nach Anspruch 33 oder 34,
**dadurch gekennzeichnet,**
**daß** eine Diagnose- oder Behandlungsstation (4) mit der Grundplatte (11) auf einer Höhe liegt und mit Führungs- und Haltebolzen (22) derart ausgestattet ist, daß dort die Patientenliege (1) nach der Querverschiebung (9) durch Endanschläge (21) positioniert ist.

36. System nach einem der Ansprüche 1 bis 35,
**dadurch gekennzeichnet,**
**daß** der Transportwagen (2) an seiner Vorderseite (35) eine portalartige Öffnung (36) aufweist, derart, daß er über eine Diagnose- oder Behandlungsstation (4) gefahren werden kann, damit diese die Patientenliege (1) mit der mindestens einen Adapterplatte (3) übernehmen kann.

37. System nach einem der Ansprüche 1 bis 36,
**dadurch gekennzeichnet,**
**daß** der Transportwagen (2) zumindest am Kopfende (23) Federungen (24) aufweist.

38. System nach einem der Ansprüche 1 bis 37,
**dadurch gekennzeichnet,**
**daß** der Transportwagen (2) eine Radbremse (25) und eine Festlegung (26) in Fahrtrichtung für die vorderen Räder (27) aufweist.

39. System nach einem der Ansprüche 1 bis 38,
**dadurch gekennzeichnet,**
**daß** mindestens ein weiteres Adapterteil (28) vorgesehen ist, welches der Anordnung der Patientenliege (1) auf einer Diagnose- oder Behandlungsstation (4) dient, für die keine der Adapterplatten (3) ausgebildet ist.

40. System nach Anspruch 39,
**dadurch gekennzeichnet,**
**daß** das Adapterteil (28) der Schaffung einer Auflage auf einer vorhandenen Patientenliege (45) dient.

41. System nach Anspruch 39 oder 40,
**dadurch gekennzeichnet,**
**daß** das Adapterteil (28) aus mindestens zwei Auflagestegen (29) besteht, die durch mindestens eine Stange (30) miteinander verbindbar sind.

42. System nach Anspruch 35 mit 39, 40 oder 41,
**dadurch gekennzeichnet,**
**daß** das Adapterteil (28) Führungs- und Haltebolzen (22) aufweist, um die Diagnose- oder Behandlungsstation (4) mit Endanschlägen (21) auszustatten.

43. System nach einem der Ansprüche 17 bis 42,
**dadurch gekennzeichnet,**
**daß** der Operationstisch (12) mit einer synchronen elektrischen Höhenverstellung (13) an allen vier Beinen (40) ausgestattet ist und daß eine Einrichtung vorgesehen ist, die nach jeder Betätigung der Höhenverstellung (13) die Stromversorgung vom Netz trennt und diese nur auf ausdrücklichen Befehl wieder herstellt.

44. System nach einem der Ansprüche 17 bis 43,
**dadurch gekennzeichnet,**
**daß** der Operationstisch (12) durch eine am Boden (31) angebrachte Standfixierung (32) unverrückbar aber abhebbar gelagert ist.

45. System nach einem der Ansprüche 17 bis 44,
**dadurch gekennzeichnet,**
**daß** der Operationstisch (12) mit vertikal (33) und horizontal (34) angeordneten Röntgenkassettenhaltem (33, 34) ausgestattet ist.

46. System nach einem der Ansprüche 1 bis 45,
**dadurch gekennzeichnet,**
**daß** die Patientenliege (1) sowie die mindestens eine Adapterplatte (3) eine Verjüngung am Kopfende (23') aufweisen.

47. System nach einem der Ansprüche 1 bis 46,
**dadurch gekennzeichnet,**
**daß** es Adapterplatten (3) beinhaltet, die an vorhandene Diagnose- oder Behandlungsstationen (4) individuell angepaßt sind.

48. System nach einem der Ansprüche 1 bis 47,
**dadurch gekennzeichnet,**
**daß** der Transportwagen (2) eine Höhenverstellung aufweist.

## Claims

1. A system for positioning and transporting a patient, comprising a transportable patient's couch top (1) and a trolley (2) for transportation of the patient's couch top (1) with the patient, said patient's couch top (1) being adapted for removal from said trolley (2) and for setup on or in a diagnostic or therapy station (4),
**characterized in that**
at least one adapter plate (3) is provided which can be assigned to said patient's couch top (1), and said patient's couch top (1), said adapter plate (3) and said trolley (2) can be interconnected and are designed in such a manner that the patient's couch top (1) can be removed, optionally separately or together with one or more adapter plates, and moved to the respective diagnostic or therapy station such that the patient's couch top (1) supporting the patient can be moved from said trolley (2) to various, differently designed diagnostic or therapy stations (4) and back.

2. A system as defined in claim 1,
**characterized in that**
said patient's couch top (1) is designed in such a manner that it can be separately moved to at least one diagnostic or therapy station (4) and back.

3. A system as defined in claim 2,
**characterized in that**
the at least one adapter plate (3) is designed in such a manner that by means thereof the patient's couch top (1) can be moved to at least one further diagnostic or therapy station (4) and back.

4. A system as defined in any one of claims 1 to 3,
**characterized in that**
at least one of the supports (5) for the patient's couch top (1) or the at least one adapter plate (3) is designed in such a manner is that it a makes it possible to convey said couch top by lifting (6) and accurately refitting (7) the same.

5. A system as defined in any one of claims 1 to 4,
**characterized in that**
at least one of the supports (5') for the patient's couch top (1) or the at least one adapter plate (3) is designed in such a manner that it makes it possible to move said couch top by displacing it in the longitudinal direction (8).

6. A system as defined in any one of claims 1 to 5,
**characterized in that**
at least one of the supports (5") for the patient's couch top (1) or the at least one adapter plate (3) is designed in such a manner that it makes it possible to move said couch top by displacing it in a transverse direction (9).

7. A system as defined in any one of claims 1 to 6,
**characterized in that**
the patient's couch top (1) and the at least one adapter plate (3) are equipped with positioning devices (10) for defined non-displaceable setups.

8. A system as defined in claim 7,
**characterized in that**
said trolley (2) is also equipped with positioning devices (10) for a defined non-displaceable setup.

9. A system as defined in any one of claims 4 to 8,
**characterized in that**
the at least one support (5, 5', 5") has means for safeguarding against unintentional lifting (6).

10. A system as defined in any one of claims 1 to 9,
**characterized in that**
the materials of its individual components (1, 2, 3) are such that there is no likelihood of disturbances being caused in the diagnostic or therapy stations (4) due to interaction with said components (1, 2, 3).

11. A system as defined in claim 10,
**characterized in that**
said trolley (2) and the at least one adapter plate (3) are made of non-magnetic or only very weakly magnetic materials.

12. A system as defined in claim 10 or claim 11,
**characterized in that**
the patient's couch top (1) is made of non-magnetic and non-conducting materials.

13. A system as defined in claim 10, 11 or 12,
**characterized in that**
said patient's couch top (1) and said adapter plate (3) supporting the same contain no metal.

14. A system as defined in any one of claims 1 to 13,
**characterized in that**
at least one of said diagnostic or therapy stations (4) is designed such that an adapter plate (3) can be set up therein in a non-displaceable manner.

15. A system as defined in any one of claims 1 to 14,
**characterized in that**
an adapter plate (3) is a base plate (11) which can be disposed non-displaceably on said trolley (2) but which can be removed therefrom by lifting.

16. A system as defined in claim 14 and 15,
**characterized in that**
said trolley (2) is designed in such a manner that it can be conveyed together with said base plate (11) over a diagnostic or therapy station (4), said system having a further support (50) and vertical adjustment means (13) such that said base plate (11) can be lifted (6) with said patient's couch top (1) from said trolley (2) and be reattached (7) thereto, and that, following lifting (6) of said base plate (11), said trolley (2) can be removed from said diagnostic or therapy station (4).

17. A system as defined in claim 14.15 or claim 16,
**characterized in that**
said diagnostic or therapy station (4) is an operating table (12).

18. A system as defined in any one of claims 1 to 17,
**characterized in that**
said patient's couch top (1) has a longitudinally displaceable support (5').

19. A system as defined in claim 18,
**characterized in that**
said longitudinally displaceable support (5') is formed in such a manner that it is not possible to lift (6) said patient's couch top (1).

20. A system as defined in claim 18 or claim 19,
**characterized in that**
a diagnostic or therapy station (4) has a support which is in the form of a counterpart to said longitudinally displaceable support (5') for said patient's couch top (1), such that said patient's couch top (1) can be pushed directly into said diagnostic or therapy station (4) from said trolley (2).

21. A system as defined in claim 20,
**characterized in that**
said longitudinally displaceable support (5') is appropriately adapted to match a support (14) in a nuclear magnetic resonance tomography apparatus (15).

22. A system as defined in claim 21,
**characterized in that**
said patient's couch top (1) is designed as a flat unit such that it can be pushed over the patient's couch top (17) of a nuclear magnetic resonance tomography apparatus (15), the support (14) in said nuclear magnetic resonance tomography apparatus (15) being positioned and designed for this purpose.

23. A system as defined in any one of claims 1 to 22,
**characterized in that**
at least one positioning device for fixing members of the body to be treated can be firmly connected to said patient's couch top (1).

24. A system as defined in claim 23,
**characterized in that**
a head holder (16) is disposed at the end of said patient's couch top (1).

25. A system as defined in claim 24,
**characterized in that**
said head holder (16) simultaneously serves as a positioning stop to terminate longitudinal displacement (8).

26. A system as defined in claim 24 or claim 25,
**characterized in that**
said head holder (16) is disposed in such a manner that despite the position of said patient's couch top (1) over the patient's couch top (17) for the nuclear magnetic resonance tomography apparatus (15) a head-holding ring (18) disposed thereon is positioned in such a manner that a head held therein comes to rest in a central region of said nuclear magnetic resonance tomography apparatus (15).

27. A system as defined in any one of claims 1 to 26,
**characterized in that**
between said patient's couch top (1) and said base plate (11) there is provided an adapter plate (3) serving as a distance plate (19).

28. A system as defined in claim 27,
**characterized in that**
said distance plate (19) has a support (5") which is transversely displaceable relatively to said base plate (11).

29. A system as defined in claim 28,
**characterized in that**
said transversely displaceable support (5") consists of guides (20) having safeguarding means against lifting (6) of said distance plate (19) and also having end stops (21) for positioning purposes.

30. A system as defined in claim 28 or claim 29,
**characterized in that**
said transversely displaceable support (5") is adapted for sliding (9) toward one side and has end stops (21) for any displacement toward the other side.

31. A system as defined in claim 28, claim 29, or claim 30,
**characterized in that**
said transversely displaceable support (5") has V-shaped guides (20) which are wide on the outside and taper toward said end stop (21).

32. A system as defined in any one of claims 28 to 31,
**characterized in that**
at least two guides (20) having end stops (21) are provided on each side.

33. A system as defined in claim 32,
**characterized in that**
said end stops (21) are produced by cooperation of the end regions of said guides (20) with guide/retainer bolts (22).

34. A system as defined in claim 33,
**characterized in that**
the distance plate (19) can be optionally displaced (9) to one or the other side due to releasably mounted guide/retainer bolts (22).

35. A system as defined in claim 33 or claim 34,
**characterized in that**
a diagnostic or therapy station (4) is at the same level as said base plate (11) and is equipped with guide/retainer bolts (22) such that said patient's couch top (1) is positioned therein by means of end stops (21) following transverse displacement (9) thereof.

36. A system as defined in any one of claims 1 to 35,
**characterized in that**
said trolley (2) has at its front end (35) a portal-like opening (36) such that it can be transported over a diagnostic or therapy station (4) so that the latter can accommodate said patient's couch top (1) together with said at least one adapter plate (3).

37. A system as defined in any one of claims 1 to 36,
**characterized in that**
said trolley (2) has resilient means (24) at at least its head end (23).

38. A system as defined in any one of claims 1 to 37,
**characterized in that**
said trolley (2) has a wheel brake (25) and locking means (26) on the front wheels (27) thereof to prevent movement in the direction of travel.

39. A system as defined in any one of claims 1 to 38,
**characterized in that**
at least one further adapter member (28) is provided which serves to position said patient's couch top (1) on a diagnostic or therapy station (4), for which none of said adapter plates (3) is designed.

40. A system as defined in claim 39,
**characterized in that**
said adapter member (28) serves to create an overlay on an existing patient's couch top (45).

41. A system as defined in claim 39 or claim 40,
**characterized in that**
said adapter member (28) consists of at least two supporting bars (29) which can be interconnected by at least one rod (30).

42. A system as defined in claim 35 in conjunction with claim 39, claim 40, or claim 41,
**characterized in that**
said adapter member (28) has guide/retainer bolts (22) in order to provide said diagnostic or therapy station (4) with end stops (21).

43. A system as defined in any one of claims 17 to 42,
**characterized in that**
said operating table (12) is equipped with synchronous electrical vertical adjustment means (13) on all four legs (40) and that means are provided which, following each actuation of the vertical adjustment means (13), cut the power supply from the mains and re-establish the power supply only on express command.

44. A system as defined in any one of claims 17 to 43,
**characterized in that**
said operating table (12) is releasably mounted on the floor (31) by stand fixing means(32).

45. A system as defined in any one of claims 17 to 44,
**characterized in that**
said operating table (12) is equipped with vertically (33) and horizontally (34) disposed X-ray casette holders (33, 34).

46. A system as defined in any one of claims 1 to 45,
**characterized in that**
said patient's couch top (1) and also said at least one adapter plate (3) exhibit a taper at the head end (23').

47. A system as defined in any one of claims 1 to 46,
**characterized in that**
it comprises adapter plates (3) which can be individually adjusted to existing diagnostic or therapy stations (4).

48. A system as defined in any one of claims 1 to 47,
**characterized in that**
said trolley (2) has vertical adjustment means.

## Revendications

1. Système de transport de patients en position allongée avec un brancard transportable (1) et un chariot de transport (2) pour le transport du brancard (1) avec un patient, le brancard (1) pouvant être retiré du chariot de transport (2) et disposé sur un poste de diagnostic ou de traitement (4),
**caractérisé en ce qu'**
il est au moins prévu une plaque adaptatrice (3) associée au brancard (1), le brancard (1), le plateau adaptateur (3) et le chariot de transport (2) étant reliés entre eux et étant réalisés de sorte que le brancard est retiré au choix séparément ou avec plusieurs plateaux adaptateurs et peut être transféré au poste respectif de diagnostique ou de traitement, de sorte que le brancard (1) avec le patient allongé peut être amené à et ramené de différents postes de traitement et de diagnostic (4).

2. Système selon la revendication 1, **caractérisé en ce que** le brancard (1) est conçu de sorte qu'il peut être amené séparément sur au moins un poste de diagnostic ou de traitement (4).

3. Système selon la revendication 2, **caractérisé en ce qu'**au moins un plateau adaptateur (3) est équipé de sorte que celui-ci peut être amené avec le brancard (1) à au moins un autre poste de diagnostic ou de traitement.

4. Système selon l'une des revendications 1 à 3, **caractérisé en ce qu'**au moins l'une des positionnements (5) du brancard respectivement d'au moins un plateau adaptateur (3) est réalisé de sorte qu'il permet un transfert par soulèvement (6) et placement ajusté (7).

5. Système selon l'une des revendications 1 à 4, **caractérisé en ce qu'**au moins l'un des positionnements (5') du brancard respectivement d'au moins un plateau adaptateur (3) est réalisé de sorte qu'il permet un transfert par déplacement dans le sens longitudinal (8).

6. Système selon l'une des revendications 1 à 5, **caractérisé en ce qu'**au moins l'un des positionnements (5") du brancard (1) respectivement d'au moins une plaque adaptatrice (3) est configuré de sorte qu'il permet un transfert par déplacement dans le sens transversal (9).

7. Système selon l'une des revendications 1 à 6, **caractérisé en ce que** le brancard (1) et au moins une plaque adaptatrice (3) sont munis de dispositifs de positionnement (10) pour des dispositions définies verrouillées.

8. Système selon la revendication 7, **caractérisé en ce que** le chariot de transport (2) est également muni de dispositifs de positionnement (10) pour une disposition définie verrouillée.

9. Système selon l'une des revendications 4 à 8, **caractérisé en ce qu'**une position (5, 5', 5") présente une sécurité contre un soulèvement involontaire (6).

10. Système selon l'une des revendications 1 à 9, **caractérisé en ce que** les matériaux de chacun des composants (1, 2, 3) sont sélectionnés de sorte que des perturbations des postes de diagnostic ou de traitement (4), avec lesquels ces composants (1, 2, 3) viennent interférer, sont exclues.

11. Système selon l'une des revendications 1 à 9, **caractérisé en ce que** le chariot de transport (2) et au moins un plateau adaptateur (3) se composent de matériaux non magnétiques ou que très faiblement magnétiques.

12. Système selon la revendication 10 ou 11, **caractérisé en ce que** le brancard (1) se compose de matériaux non magnétique ou non conducteurs.

13. Système selon la revendication 10, 11 ou 12, **caractérisé en ce que** le brancard (1) et le plateau adaptateur de support (3) ne contiennent pas de métal.

14. Système selon l'une des revendications 1 à 13, **caractérisé en ce qu'**au moins un des postes de diagnostic ou de traitement (4) est réalisé de sorte qu'un plateau adaptateur (3) est disposé de manière verrouillée.

15. Système selon l'une des revendications 1 à 13, **caractérisé en ce qu'**un plateau adaptateur (3) est un plateau de base (11) qui est disposé sur le chariot de transport de manière verrouillée tout en pouvant en être soulevé.

16. Système selon la revendication 14 et 15, **caractérisé en ce que** le chariot de transport (4) est réalisé de sorte qu'il peut être déplacé avec le plateau de base (11) sur un poste de diagnostic ou de traitement (4), celui-ci présentant un autre positionnement (50) et un réglage en hauteur (13) de sorte que le plateau de base (11) avec le brancard (1) peut être soulevé (6) du chariot de transport (2) et y être redéposé (7) et **en ce qu'**après un soulèvement (6) du plateau de base (11), le chariot de transport (2) peut être éloigné de ce poste de diagnostic ou de traitement.

17. Système selon la revendication 14, 15 ou 16, **caractérisé en ce que** ce poste de diagnostic ou de traitement (4) est une table d'opération (12).

18. Système selon l'une des revendications 1 à 17, **caractérisé en ce que** le brancard (1) présente un positionnement coulissant longitudinalement (5').

19. Système selon la revendication 18, **caractérisé en ce que** le positionnement coulissant longitudinalement (5') est réalisé de sorte qu'un soulèvement (6) du brancard (1) n'est pas possible.

20. Système selon la revendication 18 ou 19, **caractérisé en ce qu'**un poste de diagnostic ou de traitement (4) présente un positionnement qui est réalisé comme contrepartie au positionnement coulissant longitudinalement (5') du brancard (1) de sorte que le brancard (1) est insérable par le chariot de transport (2) directement dans le poste de diagnostic ou de traitement (4).

21. Système selon la revendication 20, **caractérisé en ce que** le positionnement coulissant longitudinalement (5') est adapté en conséquence avec un positionnement (14) dans un tomographe à résonance magnétique (15).

22. Système selon la revendication 21, **caractérisé en ce que** le brancard (1) est construit plat de sorte qu'il peut être poussé au-dessus du brancard (17) d'un tomographe à résonance magnétique (15), le positionnement (14) étant réalisé et disposé dans ce but dans le tomographe à résonance magnétique (15).

23. Système selon l'une des revendications 1 à 22, **caractérisé en ce qu'**au moins un dispositif de positionnement peut être relié fixement au brancard (1) pour la fixation de parties corporelles à traiter.

24. Système selon la revendication 23, **caractérisé en ce qu'**une fixation de têtière (16) est disposée à l'extrémité du brancard (1).

25. Système selon la revendication 24, **caractérisé en ce que** la fixation de têtière (16) sert en même temps de butée de positionnement à l'extrémité d'un coulissement longitudinal (8).

26. Système selon la revendication 24 ou 25, **caractérisé en ce que** la fixation de têtière (16) est disposée de sorte que malgré la situation du brancard (1), une bague de fixation de têtière (18) est positionnée au-dessus du brancard (17) du tomographe à résonance magnétique (15), de sorte qu'une tête y étant maintenue vient s'appliquer dans une zone médiane du tomographe à résonance magnétique (15).

27. Système selon l'une des revendications de 1 à 26, **caractérisé en ce qu'**entre le brancard (1) et le plateau de base (11), il est prévu un plateau adaptateur (3) conçu comme un plateau intermédiaire (19).

28. Système selon la revendication 27, **caractérisé en ce que** le plateau intermédiaire (19) présente un positionnement (5") coulissant transversalement par rapport au plateau de base (11).

29. Système selon la revendication 28, **caractérisé en ce que** le positionnement (5") coulissant transversalement se compose de guides (20) avec un verrouillage contre un soulèvement (6) du plateau intermédiaire (19) ainsi que des butées de fin de course (21) pour le positionnement.

30. Système selon la revendication 28 ou 29, **caractérisé en ce que** le positionnement (5) coulissant transversalement permet un déplacement (9) vers un côté et présente de l'autre côté des butées de fin de course (21).

31. Système selon la revendication 28, 29 ou 30, **caractérisé en ce que** le positionnement coulissant transversalement (5") présente des guides coniques (30) s'élargissant en V vers l'extérieur et se rétrécissant en direction de la buté de fin de course (21).

32. Système selon l'une des revendications 28 à 31, **caractérisé en ce qu'**il est prévu de chaque côté au moins deux guides (20) avec des butées de fin de course (21).

33. Système selon la revendication 32, **caractérisé en ce que** les butées de fin de course (21) résultent d'une collaboration des zones d'extrémité des guides (20) avec des boulons de guidage et de retenue (22).

34. Système selon la revendication 33, **caractérisé en ce que** le plateau intermédiaire (19) est coulissant au choix vers l'un ou l'autre côté par l'intermédiaire de boulons de guidage et de retenue (22) fixables et amovibles.

35. Système selon la revendication 33 ou 34, **caractérisé en ce qu'**un poste de diagnostic ou de traitement (4) se trouve à une hauteur avec le plateau de base (11) et est équipé de boulons de guidage et de retenue (22) de sorte qu'après le déplacement transversal (9), le brancard (1) y est positionné par des butées de fin de course (21).

36. Système selon l'une des revendications 1 à 35, **caractérisé en ce que** le chariot de transport (2) présente sur sa face avant (35) une ouverture en forme de portique (36) de sorte qu'il peut être déplacé au-dessus d'un poste de diagnostic ou de traitement (4) afin que celui-ci puisse recevoir le brancard (1) avec au moins un plateau adaptateur (3).

37. Système selon l'une des revendications 1 à 36, **caractérisé en ce que** le chariot de transport (2) présente au moins à la têtière (23) des ressorts (24).

38. Système selon l'une es revendications 1 à 37, **caractérisé en ce que** le chariot de transport (2) présente un frein de roulette (25) et une fixation (26) dans le sens de la marche pour les roues avant (27).

39. Système selon l'une des revendications 1 à 38, **caractérisé en ce qu'**au moins une autre pièce adaptatrice (28) est prévue laquelle sert à la disposition du brancard (1) sur un poste de diagnostic ou de traitement (4), pour lequel n'est conçu aucun des plateaux adaptateurs (3).

40. Système selon la revendication 39, **caractérisé en ce que** la pièce adaptatrice (28) sert à l'application sur un brancard existant (45).

41. Système selon la revendication 39 ou 40, **caractérisé en ce que** la pièce adaptatrice (28) se compose d'au moins deux traverses d'application (29) qui sont reliables entre elles par au moins une barre (30).

42. Système selon la revendication 35 et 39, 40 ou 41, **caractérisé en ce que** la pièce adaptatrice (28) présente des boulons de guidage et de retenue (22) pour équiper les postes de diagnostic ou de traitement (4) de butées de fin de course (21).

43. Système selon l'une des revendications 17 à 42, **caractérisé en ce que** la table d'opération (12) est munie d'un réglage en hauteur électrique synchrone (13) sur les quatre pieds (40) et **en ce qu'**il est prévu un dispositif qui coupe l'alimentation électrique du secteur après chaque actionnement du réglage en hauteur (13) et ne le rétablit qu'après instruction expresse.

44. Système selon l'une des revendications 17 à 43, **caractérisé en ce que** la table d'opération (12) est logée de manière verrouillée mais soulevable par une fixation verticale (32) placée au sol.

45. Système selon l'une des revendications 17 à 44, **caractérisé en ce que** la table d'opération (12) est munie de supports de cassettes radiographiques (33, 34) disposées horizontalement (34) et verticalement (33).

46. Système selon l'une des revendications 1 à 45, **caractérisé en ce que** le brancard (1) ainsi qu'au moins un plateau adaptateur (3) présentent un effilement à la têtière (23') .

47. Système selon l'une des revendications 1 à 46, **caractérisé en ce qu'**il contient des plateaux adaptateurs (3) qui sont adaptés individuellement aux postes de diagnostic ou de traitement (4).

48. Système selon l'une des revendications 1 à 47, **caractérisé en ce que** le chariot de transport (2) présente un réglage en hauteur.
